## Europäisches Patentamt
## European Patent Office
### Office européen des brevets

(19)

(11) Numéro de publication: **0 147 318 B1**

# (12) FASCICULE DE BREVET EUROPEEN

(45) Date de publication du fascicule du brevet:
**24.08.88**

(51) Int. Cl.⁴: **A 61 F 9/00**

(21) Numéro de dépôt: **84402679.9**

(22) Date de dépôt: **20.12.84**

(54) Appareil chirurgical pour kératotomie radiaire.

(30) Priorité: **21.12.83 FR 8320461**

(43) Date de publication de la demande:
**03.07.85 Bulletin 85/27**

(45) Mention de la délivrance du brevet:
**24.08.88 Bulletin 88/34**

(84) Etats contractants désignés:
**BE CH DE FR GB IT LI LU NL**

(56) Documents cités:
**FR - A - 2 364 646**
**FR - A - 2 490 954**
**US - A - 4 340 059**
**US - A - 4 406 285**

(73) Titulaire: **LABORATOIRE HYDRON, 6, Villa E. Bergerat, F-92200 Neuilly-sur-Seine (FR)**

(72) Inventeur: **Hanna, Khalil, 20 rue des Quatre Fils, F-75003 Paris (FR)**

(74) Mandataire: **Martin, Jean-Jacques et al, Cabinet REGIMBEAU 26, Avenue Kléber, F-75116 Paris (FR)**

ACTORUM AG

## Description

La présente invention concerne un appareil chirurgical pour kératotomie radiaire.

On entend par kératotomie radiaire une méthode chirurgicale consistant à modifier la courbure de la cornée d'un œil en pratiquant sur cette cornée des incisions radiales en référence à l'axe optique de l'œil; cette kératotomie radiaire est utilisée notamment pour réduire la courbure de la cornée dans une zone centrale ou zone optique de celle-ci, soit de façon régulière pour corriger une myopie simple, soit, lorsqu'il y a astigmatisme simple ou associé avec une myopie, respectivement de façon localisée correspondant au méridien le plus courbe de l'astigmatisme ou de façon modulée, par un choix approprié du nombre et de la localisation des incisions, de leur profondeur, et de leurs limites respectivement en rapprochement et en éloignement par rapport à l'axe optique.

Pour mettre en œuvre cette kératotomie radiaire, on a proposé antérieurement à la présente invention divers dispositifs qui, tous, mettent en œuvre un couteau unique que le chirurgien actionne manuellement pour pratiquer successivement les différentes incisions et qui ne différent que par le mode de guidage plus ou moins sophistiqué du couteau en vue de la réalisation de chacune des incisions successives.

Le plus simple de ces dispositifs de l'Art antérieur à la présente invention est décrit dans la revue «Ophtalmology» de Janvier 1983 (Volume 90 – N° 1 – PERK Study), et consiste en un couteau se composant d'un manche et d'une lame tranchante dépassant d'une garde jouant le rôle de patin d'appui du dispositif sur la cornée; la valeur de la saillie formée par la lame par rapport à la garde peut être réglée au moyen d'organes à déplacement micrométrique, et conditionne la profondeur de l'incision; les incisions sont pratiquées à tour de rôle au moyen de ce couteau, que l'on déplace le long d'un marquage préalablement réalisé sur la cornée par exemple au moyen d'un dispositif du type décrit par FR-A-2 490 954 ou par la demande de brevet FR-A-2 530 948, en partant d'une zone centrale et en direction du limbe.

La mise en œuvre d'un tel dispositif laisse une très grande part à l'habilité manuelle du chirurgien, et se caractérise par son manque de précision quelle que soit cette habilité, ne serait-ce que du fait que certaines des incisions, par exemple au nombre de 8, sont malcommodes à réaliser puisque situées à main gauche du chirurgien; en outre, le temps nécessaire à la réalisation des différentes incisions est suffisant pour que la lumière du microscope opératoire provoque une déshydratation sensible de la cornée, avec pour conséquence une variation d'épaisseur de cette dernière, difficile à prévoir et par conséquent à prendre en compte au fur et à mesure que l'on pratique les incisions successives; il existe de ce fait un risque important pour que la profondeur d'incision prévue en fonction de l'épaisseur de la cornée à l'état normal, traduite par le réglage initial de la valeur de la saillie formée par la lame par rapport à la garde, devienne excessive pour les dernières incisions réalisées, et aboutisse à une perforation de la chambre antérieure, complication grave de l'intervention.

En outre, la réalisation successive des différentes incisions au cours de l'intervention fait perdre sa rigidité à la cornée, le globe oculaire devenant hypotone, si bien que les dernières incisions sont très difficiles à réaliser.

US-A-4 340 059 décrit un dispositif très voisin, si ce n'est qu'au lieu de s'appuyer sur l'œil au moyen d'une garde formant patin d'appui, le couteau s'appuie sur une coulisse curviligne portée par une pince d'immobilisation mécanique sur l'œil et destinée à être placée dans un plan incluant l'axe optique de telle sorte que la courbure de cette coulisse soit homothétique de la courbure naturelle de la zone correspondante de la cornée; un réglage micrométrique permet de régler la profondeur d'incision mais les limites de cette dernière respectivement en rapprochement et en éloignement vis-à-vis de l'axe optique résultent d'un simple contrôle visuel; ce dispositif semble au premier abord offrir une précision indépendante de la plus ou moins grande habilité du chirurgien; cependant, le guidage coercitif du couteau dans la coulisse présentant une forme déterminée et occupant une position déterminée par rapport à l'œil considéré dans son ensemble n'autorise pas une uniformité de profondeur d'incision si l'on tient compte d'une part de ce que la cornée, préalablement à l'incision, peut présenter diverses formes qui ne sont pas nécessairement homothétiques de celle de la coulisse, et d'autre part de ce que la cornée se déforme au fur et à mesure que les incisions sont pratiquées comme on l'a dit précédemment; de ce fait, ce dispositif n'apporte en réalité pas de solution aux inconvénients du dispositif précédemment commenté.

Pour améliorer la régularité de la profondeur d'incision ainsi que la forme et le positionnement des différentes incisions, on a proposé d'associer à un couteau muni d'une garde dont fait saillie une lame tranchante un gabarit de guidage de la lame et d'appui de la garde du couteau, lequel gabarit présente une surface de référence concave propre à épouser la cornée, une surface convexe disposée à une distance déterminée, éventuellement variable, de la surface de référence pour servir d'appui à la garde du couteau, et une pluralité de fentes disposées en étoile pour autoriser le passage de la lame du couteau; la revue «Ophtalmic Surgery» (Volume 12 – N° 8 – Août 1981 – pages 561 à 566 – Kramer & Al) décrit un gabarit de ce type, dans lequel les fentes s'ouvrent dans un évidement central, alors que US-A-4 406 285 décrit un gabarit de ce type dans lequel chaque fente présente deux extrémités, limitant l'incision respectivement dans le sens d'un rapprochement vis-à-vis de l'axe optique et dans le sens d'un éloignement vis-à-vis de cet axe.

De tels gabarits présentent l'avantage d'éviter un appui direct de la garde du couteau sur l'œil et de minimiser ainsi les efforts locaux développés sur la cornée; en outre, de tels gabarits permettent d'améliorer la régularité de la profondeur d'incision, déterminée par la valeur de la saillie que la lame du couteau forme par rapport à la surface de référence, et éventuellement, par le choix d'un gabarit dont l'épaisseur est variable; de prévoir sans difficulté une variation de la profondeur d'incision par exemple suivant que l'on se rapproche ou que l'on s'éloigne de l'axe optique; cependant, le réglage de la profondeur d'incision ne peut être opéré que par changement de gabarit pour un couteau donné, ce qui oblige à tenir un stock important de gabarits; en outre, la longueur de chaque incision est imprécise si l'on ne peut utiliser, pour la déterminer, des extrémités des fentes et, du fait que les incisions sont réalisées successivement, d'une part l'intervention reste longue avec les inconvénients déjà signalés antérieurement, et d'autre part la cornée perd progressivement sa rigidité au fur et à mesure que l'on pratique les incisions si bien que les dernières incisions sont particulièrement difficiles à réaliser.

Le but de la présente invention est de proposer un appareil de kératotomie radiaire remédiant à ces inconvénients et, à cet effet, la présente invention propose un appareil comprenant un support présentant:

– un axe de support,

– une surface de référence concave, sécante de l'axe du support et propre à épouser la cornée d'un œil dans une position dans laquelle l'axe du support coïncide avec l'axe optique de l'œil,

– une pluralité de fentes dont chacune est disposée selon un plan respectif incluant l'axe du support et débouche dans une zone de la surface de référence, chaque fente autorisant le passage d'une lame de telle sorte que ladite lame présente une partie active formant une saillie déterminée par rapport à la surface de référence et puisse évoluer suivant ledit plan de la fente entre deux limites déterminées, respectivement de rapprochement et d'éloignement de la partie active de la lame par rapport à l'axe du support,

et caractérisé en ce que le support porte une pluralité de lames dont chacune est associée à une fente respective, et des moyens, disposés en retrait par rapport à la surface de référence, pour provoquer un déplacement simultané des lames par rapport au support, dans les fentes respectivement associées et selon ledit plan respectif de celles-ci, d'une même première desdites deux limites déterminées respectives à la deuxième de ces deux limites déterminées respectives alors qu'une partie active de chaque lame forme une saillie respective déterminée par rapport à la surface de référence.

Ainsi, toutes les incisions sont réalisées simultanément, si bien qu'à chaque instant de l'intervention, les parties actives des différentes lames rencontrent une résistance mécanique identique de la cornée, et que les conditions mécaniques d'incision sont les mêmes pour toutes les incisions; en outre, la durée d'intervention nécessaire à la réalisation de l'ensemble des incisions voulues est considérablement réduite, ce qui est avantageux en soi et élimine en outre tout risque de déshydratation de la cornée sous la lumière du microscope opératoire ainsi que ses conséquences.

Il en résulte une grande précision géométrique des incisions, quelle que soit d'ailleurs la position de ces dernières par rapport à la main du chirurgien, et une réduction considérable de tous les risques, notamment de perforation, liés à la variation progressive d'épaisseur de la cornée, par déshydratation, et de consistance de cette cornée, par affaiblissement progressif, que l'on rencontre dans les techniques traditionnelles de réalisation successive des incisions.

De préférence, lesdites première et deuxième limites sont respectivement la limite en rapprochement et la limite en éloignement de la partie active de chaque lame par rapport à l'axe du support, ce qui signifie que les parties actives des lames réalisent les différentes incisions en s'éloignant de l'axe optique, en travaillant en traction par rapport à une zone centrale de la cornée; cependant, on peut également pratiquer l'incision par un déplacement inverse des parties actives des lames, notamment lorsque, selon un mode de réalisation préférée de l'appareil, ce dernier comporte des moyens de plaquage pneumatique de la cornée contre la surface de référence, ce qui supprime tout risque de défaut d'incision dû à l'asphéricité éventuelle et à l'élasticité de la cornée, et d'autre part assure un maintien efficace de l'appareil et de la cornée en position relative; à cet effet, avantageusement, le support délimite de façon étanche, autour des lames, un volume ouvert exclusivement vers la surface de référence notamment via lesdites fentes et comporte des moyens de raccordement dudit volume à une source d'aspiration, ce qui permet de neutraliser la résistance de la cornée à la pénétration des lames et d'assurer une reproductibilité de la profondeur de l'incision.

La position relative voulue de l'appareil et de la cornée peut être obtenue plus facilement, par approximations successives, préalablement à la réalisation des incisions, lorsque l'appareil selon l'invention présente en outre des moyens permettant d'escamoter de façon réversible, à cet effet, la partie active des lames par rapport à la surface de référence; la visibilité à travers l'appareil, au microscope opératoire, notamment lors du positionnement de l'appareil sur la cornée préalablement à la réalisation des incisions peut être améliorée sensiblement lorsque, selon un mode de réalisation préféré, la surface de référence comporte une face d'une lentille formant partie intégrante du support et présentant lesdites fentes; naturellement, cette lentille facilite le centrage en vue duquel elle présente avantageusement des moyens de repérage, tels qu'un réticule central et un cercle de diamètre prédéterminé, et la surveillance de la bonne réalisation des incisions.

Un même appareil selon l'invention peut être utilisé pour réaliser des incisions de caractéristiques géométriques différentes lorsque, comme il est préféré, des moyens sont prévus pour régler la saillie de la partie active des lames par rapport à la surface de référence, ce qui permet de régler la profondeur des incisions, ainsi que pour régler la position, en écartement par rapport à l'axe du support, des première et deuxième limites précitées, c'est-à-dire la position de chaque partie active de lame par rapport à l'axe du support respectivement au début et en fin d'incision; on remarquera que le trajet des parties actives des lames de même que la géométrie de la surface de référence, avantageusement interchangeable au moins quant à sa zone dans laquelle débouchent les fentes, peuvent être choisis de telle sorte que la profondeur d'incision soit constante ou varie de façon prédéterminée en fonction de l'écartement vis-à-vis de l'axe du support, avec lequel se confond l'axe du support; notamment, la surface de référence peut présenter une convexité limitée à une zone annulaire située autour de l'axe du support et dans laquelle débouchent les fentes, la présence de moyens de plaquage pneumatique de la cornée contre la surface de référence permettant de faire épouser sans difficulté cette convexité à la cornée, dont la courbure normale convexe s'inverse alors localement.

Enfin, les lames sont avantageusement amovibles et interchangeables, ce qui permet notamment d'utiliser des lames régulièrement réparties angulairement autour de l'axe du support, et de corriger ainsi une myopie, ou d'utiliser des lames localisées dans des secteurs angulaires déterminés, en référence à l'axe du support, pour corriger un astigmatisme; dans l'un et l'autre cas, les caractéristiques géométriques des incisions pratiquées par les différentes lames peuvent être réglées grâce aux différents moyens de réglage précités, ou encore par l'association aux différentes fentes de lame présentant elles-mêmes des caractéristiques géométriques différentes et par le choix d'une surface de référence de géométrie appropriée dans sa zone dans laquelle débouchent les fentes.

Ces possibilités sont offertes dans leur ensemble par un appareil unique, facile à utiliser, et permettant de pratiquer des incisions dans des conditions reproductibles, c'est-à-dire offrant une meilleure prévisibilité du résultat avec des chirurgiens différents.

D'autres caractéristiques et avantages de l'appareil selon l'invention ressortiront de la description ci-dessous, relative à deux modes de réalisation non limitatifs, ainsi que des dessins annexés qui font partie intégrante de cette description.

– La figure 1 montre un premier mode de réalisation d'un appareil selon l'invention, pour moitié en élévation latérale et pour moitié en coupe par un plan incluant l'axe du support, et repéré en I–I à la figure 2.

– La figure 2 montre une vue de dessus de l'appareil illustré à la figure 1, c'est-à-dire une vue suivant l'axe du support.

– La figure 3 montre une vue de l'appareil en coupe par un plan perpendiculaire à l'axe du support, repéré en III–III à la figure 1.

– La figure 4 montre un deuxième mode de réalisation de l'appareil selon l'invention, pour partie en élévation latérale et pour partie en coupe par un plan incluant l'axe du support.

– La figure 5 illustre un détail du montage d'une lame de l'appareil de la figure 4, en une vue en coupe par un plan repéré en V–V à cette figure.

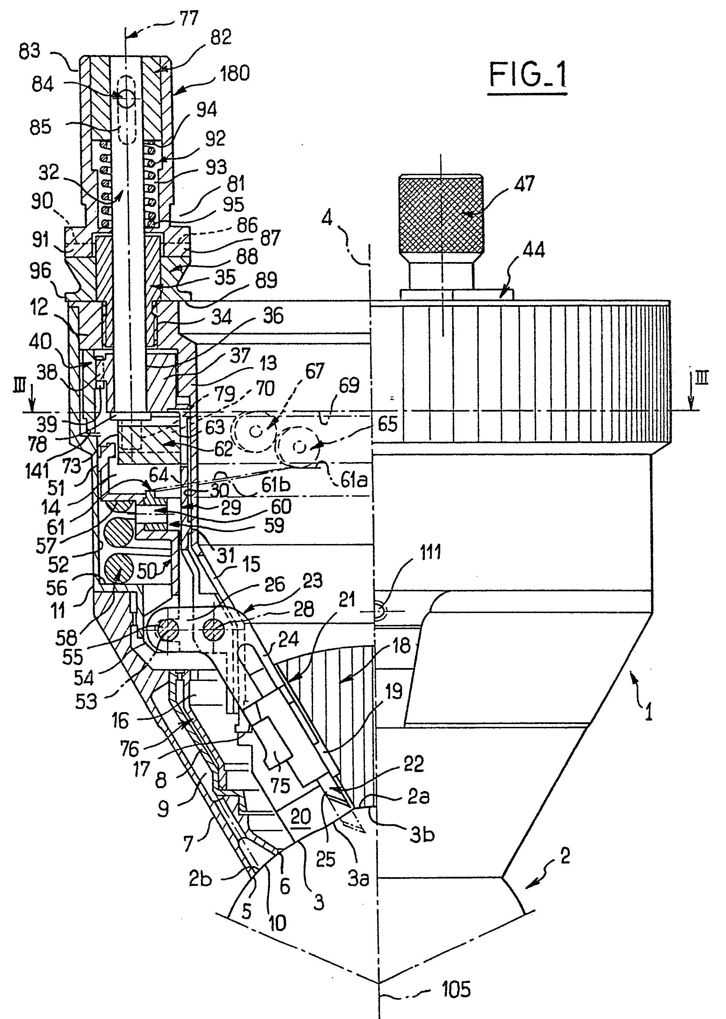

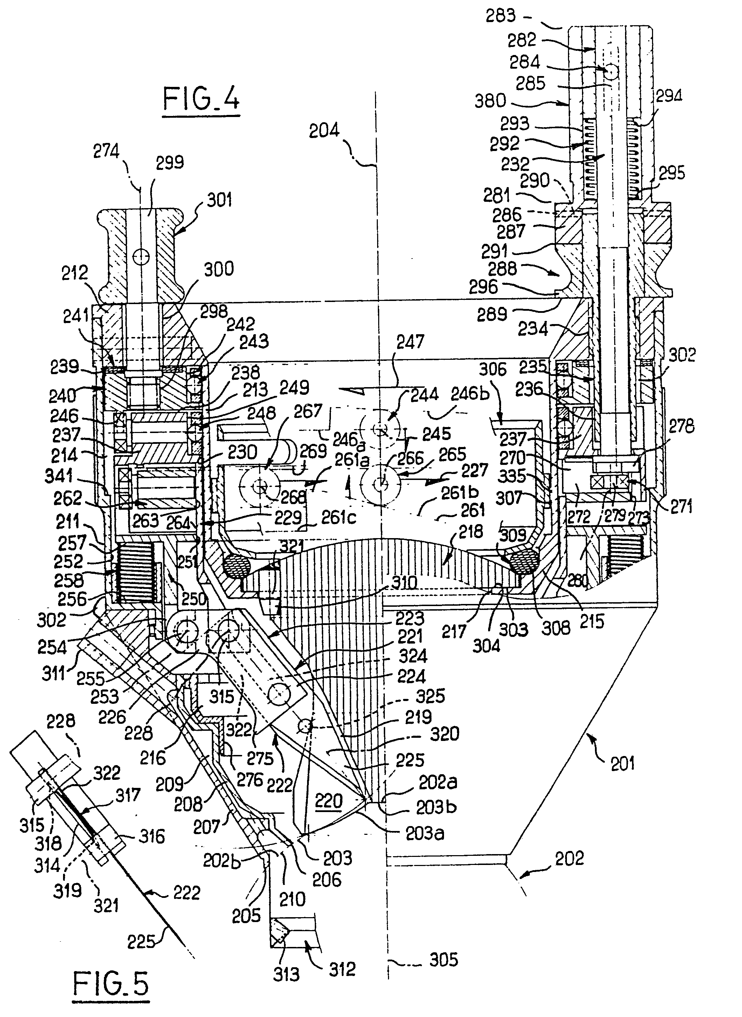

On remarquera que les figures 1 et 4 montrent l'appareil dans une position proche de sa position normale d'utilisation sur l'œil d'un patient supposé couché; toute notion d'orientation ou de niveau relatif apparaissant dans la suite de la description s'entendra par référence à cette position normale d'utilisation telle qu'elle est illustrée aux figures 1 et 4.

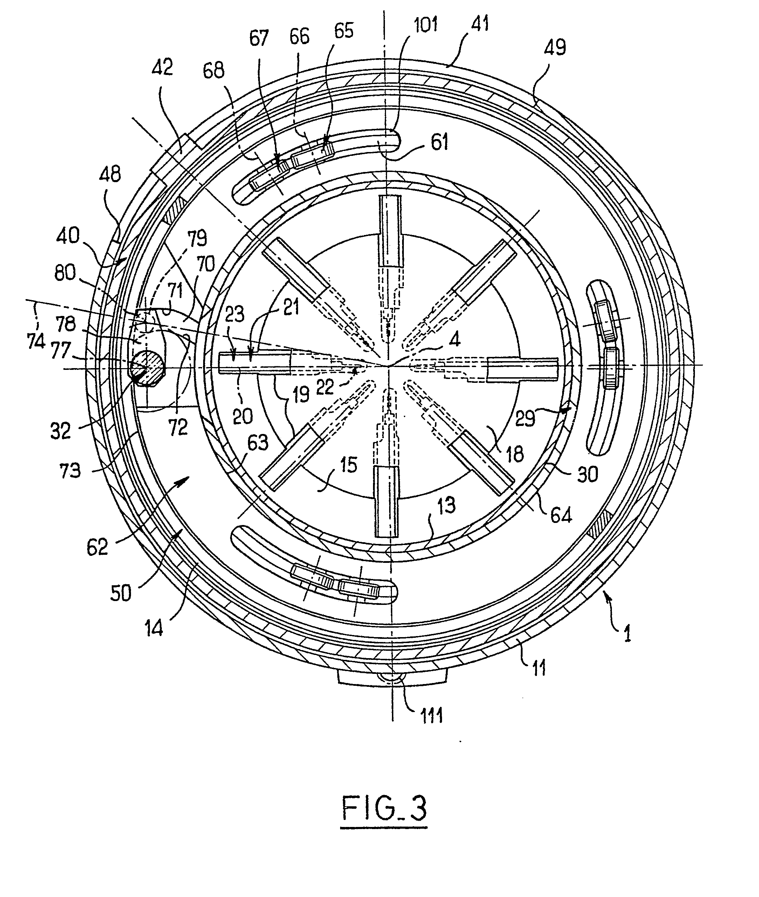

On se reportera en premier lieu aux figures 1 à 3, et plus particulièrement à la figure 1 où l'on voit que l'appareil selon l'invention comporte un corps 1, formant support, par lequel l'appareil peut être saisi et manipulé, et être posé sur la cornée 2a d'un œil 2 par une surface de référence 3 de forme concave, propre à épouser cette cornée 2a ainsi que la sclère juxta-limbique 2b dans une position dans laquelle un axe 4 du support 1 coïncide avec l'axe optique 105 de l'œil; dans l'exemple illustré, la surface de référence 3, tournée vers le bas en position d'utilisation de l'appareil, présente la forme générale d'une chalotte sphérique centrée sur l'axe 4 et sécante de celui-ci, à l'exception d'une zone localisée 3a quant à elle convexe, annulaire de révolution autour de l'axe 4, qui sera décrite plus loin; toutefois, d'autres formes de la surface de référence 3 pourraient être adoptées sans que l'on sorte pour autant du cadre de la présente invention.

Dans une zone périphérique radialement extérieure, en référence à l'axe 4, la surface de référence 3 est définie par les bords respectifs 5, 6, l'un et l'autre annulaires de révolution autour de l'axe 4, de deux parois coaxiales, respectivement 7 et 8, respectivement radialement extérieure et radialement intérieure en référence à l'axe 4; ces parois 7 et 8 délimitent entre elles, de façon étanche, un volume 9 présentant dans la surface de référence 3, entre les bords 5 et 6, une embouchure 10 annulaire, continue, de révolution autour de l'axe 4; le volume 9 peut être relié, par un raccord approprié 111 de la paroi 7, à une source de vide non représentée, qui permet de créer à l'embouchure 10 du volume 9 une dépression assurant un plaquage de la sclère juxta-limbique 2b contre la surface de référence 3, que la cornée 2a épouse ainsi au mieux.

Les deux parois 7 et 8 sont constituées de deux pièces distinctes, de forme générale tronconique de révolution autour de l'axe 4 est s'évasant de bas en haut, ces pièces étant solidarisées mutuellement de façon étanche par exemple par vissage et collage.

Vers le haut, la pièce définissant la paroi 7 se raccorde de façon étanche, solidaire mais amovible, et par exemple par vissage, à une pièce définissant quant à elle une paroi 11 de forme générale cylindrique de révolution autour de l'axe 4; cette pièce se raccorde elle-même vers le haut, de façon étanche, solidaire et amovible et par exemple par emmanchement dur, à une paroi 12, annulaire de révolution autour de l'axe 4 et située radialement à l'intérieur de la paroi 11 en référence à cet axe, d'une pièce présentant en outre une paroi 13 de forme générale cylindrique de révolution autour de l'axe 4 avec un diamètre maximal inférieur au diamètre minimal de la paroi 11 de telle sorte que la paroi 13 soit placée coaxialement à l'intérieur de cette paroi 11 et que les parois 11 et 13 délimitent entre elles un volume 14 fermé vers le haut par la paroi 12, à laquelle la paroi 13 se raccorde vers le haut; vers le bas, à un niveau supérieur à celui du raccordement des parois 11 et 7, la paroi 13 se raccorde à une paroi 15 qui est réalisée d'une pièce avec cette paroi 13 ainsi que la paroi 12 et qui présente quant à elle une forme tronconique de révolution autour de l'axe 4 et convergeant de haut en bas, en laissant subsister entre elle et les parois 7 et 8 un volume 16; la paroi 15 s'interrompt vers le bas par un bord annulaire 17, de révolution autour de l'axe 4, à un niveau supérieur à celui du bord 6 de la paroi 8, lui-même situé à un niveau supérieur à celui du bord 5 de la paroi 7 compte tenu de la forme générale concave de la surface de référence 3, au-dessus de laquelle le bord 17 est situé alors que les bords 5 et 6 participent à la définition de cette surface.

La paroi 15 porte de façon solidaire, mais de préférence amovible grâce à des moyens non représentés mais aisément concevables par l'Homme du métier, une lentille 18 de forme générale de révolution autour de l'axe 4 et qui définit les zones de la surface de référence 3 qui sont situées radialement vers l'intérieur, en référence à l'axe 4, par rapport au bord 6 de la paroi 8 lui-même situé radialement vers l'intérieur, en référence à l'axe 4, par rapport au bord 5 de la paroi 7; notamment, la lentille 18 définit la zone annulaire 3a de la surface de référence 3 avantageusement munie d'un cercle de repérage d'axe 4, ainsi qu'une zone centrale 3b de cette dernière, laquelle zone centrale 3b coupe l'axe et présente avantageusement un réticule de centrage à son intersection avec cet axe 4; on remarquera que la lentille 18 est totalement dégagée, vers le haut, par l'ensemble des parois 15, 13, 12, 11, 8, 7, si bien qu'il est possible de voir à travers l'appareil suivant l'axe 4 jusqu'au bord 6 de la paroi 8.

Dans la zone 3a de la surface de référence 3 débouchent plusieurs fentes telles que 19, aménagées dans la lentille 18 et dans la paroi 15 selon des plans tels que 20 dont chacun inclut l'axe 4 du support; dans l'exemple illustré, ces plans tels que 20, et avec eux les fentes 19, sont régulièrement répartis angulairement autour de l'axe 4 et au nombre de 8, mais d'autres dispositions relatives et d'autres nombres pourraient également être choisis sans que l'on sorte pour autant du cadre de la présente invention.

Chacune des fentes 19 est susceptible de recevoir, avec possibilité de débattement relatif suivant son plan 20, un couteau respectif 21 formé d'une lame 22 tournée vers le bas, et d'un porte-lame 23 en forme de L, présentant deux bras mutuellement solidaires dont l'un 24 est tourné vers le bas, creux, pour recevoir de façon solidaire mais amovible, par exemple par emboîtement et pincement élastique, une zone d'extrémité supérieure de la lame 22 présentant par ailleurs en saillie sous ce bras 24 une zone d'extrémité inférieure ou zone active 25, et d'un bras 26 auquel le bras 24 se raccorde vers le haut et qui est quant à lui orienté dans le sens d'un éloignement vis-à-vis de l'axe 4, approximativement radialement en référence à celui-ci; les deux bras 24 et 26 de même que la lame 22 sont disposés suivant le même plan moyen 20 que la fente 19 à laquelle ils sont associés; on remarquera qu'à chaque fente 19 est associé un porte-lame 23 auquel peut être associée une lame respective 22, étant bien entendu, que, à l'utilisation de l'appareil, certains porte-lame 23 peuvent rester démunis de lame, si bien que l'appareil peut être utilisé avec une seule lame, ou avec un nombre de lames correspondant au nombre de fentes, c'est-à-dire 8 dans l'exemple non limitatif illustré, ou encore avec tout nombre intermédiaire de lames dans des positions choisies à volonté parmi les positions des différents plans 20.

Pour l'essentiel, chaque porte-lame 23 est placé à l'intérieur du volume 16 et s'articule à l'intérieur de ce volume, à la jonction de ses deux bras 23 et 26, autour d'un axe 28 perpendiculaire au plan 20 de la fente 19 associée, sur une chape d'un chariot annulaire 29 présentant la forme générale d'un manchon cylindrique de révolution autour de l'axe 4; les axes d'articulation 28 des différents porte-lame 23 sur ce chariot 29 sont placés dans un même plan perpendiculaire à l'axe 4 et tangents, par leur point d'intersection avec les plans 20 respectifs, à un même cercle virtuel 27 d'axe 4 dans l'exemple illustré, mais d'autres dispositions relatives de ces axes et d'autres formes du chariot 29 pourraient être adoptées sans que l'on sorte pour autant du cadre de la présente invention.

Le chariot 29 enveloppe extérieurement la paroi 13 sur laquelle il est monté au coulissement relatif, de façon à pouvoir accomplir par rapport à elle, c'est-à-dire par rapport au support 1, un mouvement de translation parallèlement à l'axe 4 sans pouvoir toutefois accomplir un mouvement de rotation autour de cet axe 4 par rapport à la paroi 13 et au support 1 dans sa généralité.

A cet effet, par exemple, la paroi 13 reçoit le chariot 29 par une face 30, radialement extérieure en référence à l'axe 4, cylindrique de révolution autour de cet axe 4 et le chariot 29 est en contact avec cette face 30 par des portées annulaires 31, radialement intérieures en référence à l'axe 4, cylindriques de révolution autour de cet axe 4 avec un diamètre sensiblement identique à celui de la face 30; une rotation du chariot 29 par rapport à la paroi 13 autour de l'axe 4 est empêchée par clavetage, au moyen d'une tige 32 qui traverse parallèlement à l'axe 4, avec possibilité de coulissement relatif parallèlement à cet axe, la paroi 12 via un alésage 34 de cette dernière, lui-même muni intérieurement d'un manchon 35 de guidage de la tige 32 au coulissement parallèlement à l'axe 4, laquelle tige 32 s'engage, via un alésage 36 parallèle à l'axe 4, dans un rebord 37, annulaire de révolution autour de l'axe 4, que le chariot 29 présente en monobloc vers le haut et radialement vers l'extérieur en référence à l'axe 4, à l'intérieur du volume 14.

Radialement vers l'extérieur en référence à l'axe 4, le rebord 37 du chariot 29 présente un filetage 38 d'axe 4, en prise avec un taraudage complémentaire 39 que présente, radialement vers l'intérieur en référence à l'axe 4, une couronne annulaire 40, l'axe 4, logée à l'intérieur du volume 14 avec possibilité de rotation autour de l'axe 4 par rapport au support 1, mais sans possibilité de translation par rapport à ce support 1 parallèlement à l'axe 4; par exemple, à cet effet, la couronne 40 est en butée vers le haut contre la paroi 12 et vers le bas contre un épaulement plan 141, annulaire de révolution autour de l'axe 4, que la paroi 11 présente à l'intérieur du volume 14; le guidage de cette couronne 40 à la rotation autour de l'axe 4 vis-à-vis du support 1 est avantageusement assuré par l'intermédiaire du chariot 29, du fait de la prise mutuelle du filetage 38 de ce dernier et du taraudage 39 de la couronne 40.

En regard de la couronne 40, radialement en référence à l'axe 4, la paroi 11 est percée d'une lumière 41 située suivant un plan moyen perpendiculaire à l'axe 4, et dans laquelle s'engage un doigt 42 porté de façon solidaire par la couronne 40 pour constituer un moyen de préhension manuelle et de manœuvre de la couronne 40 à la rotation autour de l'axe 4 par rapport au support 1, c'est-à-dire également par rapport au chariot 29 qui, quant à lui immobilisé à la rotation par rapport au support 1, subit lors d'une telle rotation une translation parallèlement à l'axe 4 par rapport à ce support, dans un sens fonction du sens de rotation du doigt 42 autour de l'axe 4 par rapport au support 1 et avec une amplitude fonction de l'amplitude de cette rotation.

Dans la mesure où chacun des porte-lame 23, et avec lui la lame 22 éventuellement associée, se déplace dans une telle translation conjointement avec le chariot 29, on peut ainsi amener, par une rotation appropriée de la couronne 40 par manœuvre du doigt 42, les zones actives 25 des différentes lames 22 engagées dans les porte-lames 23 soit dans une position d'escamotage, illustrée en traits pleins à la figure 1, dans laquelle chaque zone active 25 d'une lame 22 est placée en retrait par rapport à la surface de référence 3, dans la fente 19 correspondante, ou bien dans une position, illustrée en traits mixtes à la figure 1, dans laquelle la zone active 25 de chaque lame 22 forme une saillie par rapport à la surface de référence 3, dans la zone 3a de cette dernière, la valeur de cette saillie étant fonction de la position angulaire du doigt 42 par rapport au support 1, en référence à l'axe 4.

Pour permettre de visualiser, par la position du doigt 42 par rapport au corps 1, l'escamotage des zones actives 25 des lames 22 par rapport à la surface de référence 3, ou la saillie éventuellement formée par ces zones actives 25 par rapport à cette surface de référence 3 ainsi que la valeur de cette saillie éventuelle, la paroi 12 du support 1 présente extérieurement, à proximité de la lumière 41, une graduation 43 représentative de la position de la zone active 25 des lames 22 par rapport à la surface de référence 3 et, lorsqu'il y a saillie des zones actives 25 des lames 22 par rapport à cette surface de référence 3, de la valeur de cette saillie qui elle-même conditionne la profondeur d'incision.

En outre, des moyens sont prévus pour limiter cette profondeur d'incision, c'est-à-dire la valeur de la saillie formée éventuellement par les parties actives 25 des lames 22 par rapport à la surface de référence 3, sous la forme d'un coulisseau 44 porté par la paroi 12, par l'intermédiaire d'une lumière 45 d'axe 4 aménagée dans celle-ci à proximité de la lumière 41; ce coulisseau 44 comporte une zone 46 susceptible de former butée pour le doigt 42 lors d'une rotation de celui-ci, par rapport au support 1, dans un sens correspondant à la descente du chariot 29, c'est-à-dire à la formation d'une saillie par les zones actives 25 des lames 22 par rapport à la surface de référence 3, et un bouton 47 de manœuvre de ce coulisseau 44 au coulissement le long de la lumière 45 et de verrouillage amovible de ce coulisseau 44 sur le support 1 dans une position voulue le long de la lumière 45, par exemple par serrage contre la paroi 12.

La limitation du mouvement de translation du chariot 29 par rapport au support 1, parallèlement à l'axe 4, dans le sens d'un escamotage des zones actives 25 des lames 22 peut quant à elle être assurée soit par venue du rebord 37 du chariot 29 en butée vers le haut contre la paroi 12, soit par venue du doigt 42 en butée contre l'une 48 des extrémités 48, 49 de la lumière 41.

A titre d'exemple non limitatif, on peut ainsi prévoir une possibilité de translation du chariot 29 par rapport au support 1, entre deux limites correspondant respectivement à un escamotage de la partie active 25 des lames 22 supposées standardisées, par rapport à la surface de référence 3, d'une distance de 1 dixième de millimètre et à une saillie de cette partie active 25, par rapport à la surface de référence 3, d'une distance de 9 dixièmes de millimètres, ces limites étant repérées par des indications numériques de la graduation 43 portant également indication de la saillie correspondant à des positions intermédiaires entre ces limites.

Afin que chaque position du chariot 29 par rapport au support 1, à la translation parallèlement à l'axe 4, constitue une position stable, la prise mutuelle du filetage 38 et du taraudage 39 assure une transmission de mouvement irréversible.

Le mode de déplacement du chariot 29 par rapport au support 1 qui vient d'être décrit, supposant une translation sans rotation du chariot 29 par rapport au support 1, convient dans le cas fréquent où, comme il est illustré, la zone active 25 de chaque lame 22 est située dans un plan qui coïncide avec le plan 20 de la fente 19 associée.

Cependant, on peut également prévoir que la zone active 25 de chaque lame 22 soit située dans un plan qui forme un angle déterminé, identique d'une lame à l'autre, par rapport au plan 20 de la fente 19 associée; dans ce cas, la solidarisation du rebord 37 du chariot 29 à l'encontre de toute rotation autour de l'axe 4 par rapport au support 1, au moyen de la tige 32 traversant sans jeu le manchon 35 et l'alésage 36 du rebord 37, est supprimée et remplacée par une liaison mutuelle du chariot 29 et du support 1, et plus précisément de la paroi 13 de celui-ci, par une surface de came héliocoïdale axée sur l'axe 4 du support 1 et présentant un angle d'hélice compatible avec l'angle que forme le plan de chaque zone active 25 d'une lame 22 par rapport au plan 20 de la fente 19 associée, de telle sorte que la translation du chariot 29 par rapport au support 1 parallèlement à l'axe 4, par manœuvre du doigt 42, s'accompagne d'une rotation du chariot 29 autour de l'axe 4 par rapport au support 1, avec une amplitude telle que le plan de la zone active 25 de chaque lame 22 reste fixe par rapport au support 1; l'Homme du métier peut aisément réaliser un appareil selon l'invention d'une telle conception, qui n'a pas été illustrée, en prévoyant une possibilité de débattement angulaire de la tige 32 par rapport au support 1, autour de l'axe 4, le guidage sans jeu de la tige 32 dans l'alésage 36 du rebord 37 du chariot 29 étant conservé en raison des fonctions de la tige 32 qui apparaîtront plus loin; en outre, on peut être dans ce cas amené à évaser les fentes 19 au voisinage de la surface de référence 3.

Outre les moyens permettant de déplacer le chariot 29, et avec lui les lames 22, à la translation parallèlement à l'axe 4 par rapport au support 1, l'appareil selon l'invention comporte des moyens pour manœuvrer à volonté l'ensemble des portes-lame 23 au pivotement par rapport au chariot 29, autour des axes 28 respectifs, suivant les plans 20 des fentes 19 respectivement associées.

A cet effet, l'appareil illustré aux figures 1 à 3 comporte, à l'intérieur des volumes 14 et 16, un manchon annulaire 50 disposé coaxialement au chariot 29, radialement à l'extérieur de celui-ci en référence à l'axe 4, des moyens étant prévus pour guider ce manchon 50 à la translation par rapport au chariot 29 parallèlement à l'axe 4 sans possibilité de rotation relative autour de cet axe.

Par exemple, le guidage du manchon 50 à la translation par rapport au chariot 29 parallèlement à l'axe 4 est assuré par contact de portées annulaires telles que 51, cylindriques de révolution autour de l'axe 4 et situées radialement à l'extérieur du manchon 50 en référence à cet axe, et d'une face 52, radialement intérieure en référence à l'axe 4, que la paroi 11 présente en vue du glissement de ces portées annulaires telles que 51; l'immobilisation du manchon 50 à la rotation par rapport au chariot 29 autour de l'axe 4 est quant à elle assurée, dans l'exemple de réalisation de l'appareil qui est illustré aux figures 1 à 3, par une liaison du manchon 50, vers le bas, avec chacun des porte-lame 23 par l'intermédiaire de moyens de liaison cinématique associant, à une translation du manchon 50 par rapport au chariot 29 dans l'un ou l'autre de deux sens opposés parallèles à l'axe 4, un pivotement simultané de l'ensemble des porte-lames 23 par rapport au chariot 29, autour des axes 28, de telle sorte que les zones actives 25 des lames 22 se déplacent respectivement en rapprochement ou en éloignement par rapport à l'axe 4 du support 1, dans les plans 20 des fentes 19 associées.

Dans l'exemple illustré aux figures 1 à 3, ces moyens de liaison cinématique sont constitués sur chaque porte-lame 23 par un tourillon respectif 55 solidaire du bras 26 du porte-lame 23 dans une zone située plus loin de l'axe 4 que l'axe 28 d'articulation de ce porte-lame 23 sur le chariot 29, ce tourillon 55 présentant un axe 53 parallèle à l'axe 28; sur le manchon 50, ces moyens de liaison cinématique comportent, pour chaque porte-lame 23, une partie inférieure en étrier 54 ouverte vers l'axe 4 et susceptible de prendre appui sur le tourillon 55, sans jeu, respectivement par le haut et par le bas alors qu'elle laisse subsister vis-à-vis du tourillon 52 un jeu radial, en référence à l'axe 4.

On conçoit que, par effet de levier, une translation du manchon 50 parallèlement à l'axe 4 par rapport au chariot 29 provoque un pivotement des porte-lame 23 et des lames 22, à l'unisson, par rapport au chariot 29 autour des axes 28.

Pour permettre de provoquer à volonté un tel pivotement, l'appareil illustré aux figures 1 à 3 comporte des moyens pour manœuvrer à volonté le manchon 50 à la translation par rapport au chariot 29 dans un sens ou dans l'autre, parallèlement à l'axe 4.

A cet effet, la paroi 11 présente un épaulement inférieur 56 annulaire, plan, de révolution autour de l'axe 4, lequel épaulement 56 est tourné vers le haut, et le manchon 50 présente en regard de cet épaulement 56 un épaulement annulaire, plan, 57, également de révolution autour de l'axe 4 mais quant à lui tourné vers le bas, et entre ces épaulements 56 et 57 est intercalé un ressort hélicoïdal de compression 58, d'axe 4 sollicitant élastiquement le manchon 50 à la translation vers le haut, par rapport au chariot 29, parallèlement à l'axe 4.

En outre sont prévus des moyens réversibles de manœuvre, à volonté, du manchon 50 à la translation par rapport au manchon 29 parallèlement à l'axe 4 dans un sens allant à l'encontre du sens d'action du ressort 58.

Sur le manchon 50, ces moyens réversibles de manœuvre comportent une pièce annulaire 59 solidarisée avec le manchon 50 par tout moyen approprié, et par exemple par des goupilles telles que 60; vers le haut, cette pièce 59 définit au moins un chemin de came solidaire du manchon 50 et pentu par rapport à l'axe 4; plus précisément, dans l'exemple illustré, la pièce annulaire 59 présente une face supérieure annulaire 61 de génératrices perpendiculaires à l'axe 4, cette face 61 comportant trois tronçons identiques 61a plans, coplanaires et perpendiculaires à l'axe 4, avec lesquels alternent trois tronçons identiques 61b, régulièrement répartis angulairement autour de l'axe 4, dont chacun présente une forme hélicoïdale, en creux par rapport aux tronçons plans 61a.

Sur le chariot 29, ces moyens comportent une bague 62, de révolution autour de l'axe 4 et en contact glissant, par une face 63 cylindrique de révolution autour de l'axe 4 et radialement intérieure en référence à cet axe 4, avec une face 64, également cylindrique de révolution autour de l'axe 4 avec un diamètre identique à celui de la face 63, que le chariot 29 présente dans le sens d'un éloignement radial vis-à-vis de l'axe 4, de telle sorte que la bague 62 soit guidée à la rotation autour de l'axe 4 par rapport au manchon 29; vers le bas, en regard de la face supérieure 61 de la pièce 59 et plus précisément en regard de chacune des zones 61b de cette face, la bague 62 porte un toucheau de came sous la forme d'un galet respectif 65 monté à la rotation sur elle autour d'un axe 66 radial en référence à l'axe 4; vers le haut, la bague 62 porte une pluralité de galets 67 tourillonnant sur elle autour d'axes respectifs 68 radiaux en référence à l'axe 4; elle prend appui vers le haut, via ces galets 67, sur une face annulaire plane 68, de révolution autour de l'axe 4 et perpendiculaire à celui-ci, que le rebord 37 du chariot 29 présente vers le bas; en sollicitant le manchon 50 vers le haut par rapport au chariot 29, parallèlement à l'axe 4, le ressort 58 assure un contact permanent de la face 61 de la pièce 59 avec les galets 65 et des galets 67 de la bague 62 avec la face 69 du rebord 37 si bien que, en raison de la forme de cette face 69, la bague 62 est immobilisée à l'encontre d'une translation par rapport au chariot 29, parallèlement à l'axe 4.

L'angle d'hélice des zones 61b de la face 61 de la pièce 59 et l'aptitude des galets 65 et 67 à la rotation autour de leurs axes respectifs par rapport à la bague 62 sont choisis tels que la seule action du ressort 58 ramène, si elle n'est pas contrebalancée, le manchon 50 dans une position haute limite par rapport au manchon 29, par translation relative parallèlement à l'axe 4 et moyennant une rotation de la bague 62 du fait de l'appui des galets 65 vers le haut contre les parties pentues 61b de la face 61 de la pièce 59; cette position haute limite correspond à une position non illustrée dans laquelle chacun des galets 65 est au contact d'une zone inférieure de la partie associée 61b de la face 61 de la pièce 59, et dans laquelle la zone active 25 de chacune des lames 22 occupe sa position la plus éloignée possible de l'axe 4, compte tenu de la liaison cinématique entre le manchon 50 et chacun des porte-lames 23.

On conçoit qu'ainsi, sous l'action du ressort 58, les zones actives 25 des lames soient susceptibles de se déplacer automatiquement dans le sens d'un éloignement radial vis-à-vis de l'axe 4, jusqu'à une limite définie par butée des zones inférieures 75 des bras 24 des porte-lame 23, situées sous les axes de pivotement 28 de ces derniers, contre un manchon 76 rapporté de façon amovible, par exemple par vissage, sur la paroi 8 dans une position radialement intérieure à celle-ci, en référence à l'axe 4; l'interchangeabilité du manchon 76 permet de choisir à volonté un manchon déterminé parmi une gamme de tels manchons présentant des caractéristiques dimensionnelles différentes, en fonction de la limite que l'on veut imposer à l'éloignement de la zone active 25 de chaque lame 22 par rapport à l'axe 4, et de monter le manchon 76 ainsi choisi sur l'appareil pour offrir une butée appropriée à la zone 75 de chaque porte-lame 23.

Un pivotement de chaque porte-lames 23 autour de son axe 28 par rapport au manchon 29 dans un sens correspondant, pour la zone active 25 de chaque lame 22, à un rapprochement par rapport à l'axe 4 peut quant à lui être obtenu par une manœuvre manuelle, grâce à des moyens susceptibles de ne s'opposer que provisoirement à l'action du ressort 58, c'est-à-dire réversibles.

Ces moyens comportent notamment la tige 32, qui présente une forme générale cylindrique de révolution autour d'un axe 77 parallèle à l'axe 4, et fixe par rapport au support 1 du fait d'une coaxialité, sans jeu, de la tige 32, du manchon 35 et de l'alésage 36 définis plus haut.

A l'intérieur du volume 14, la tige 32 présente immédiatement en dessous de la face 69 du rebord 37 du chariot 29 une extrémité inférieure dont est solidaire une manivelle 78 perpendiculaire à l'axe 77, et qui elle-même porte vers le bas, de façon solidaire, suivant un axe 79 parallèle à l'axe 77 et décalé par rapport à celui-ci, un doigt 80 cylindrique de révolution autour de l'axe 79 et qui s'engage dans une gorge 70 que présente, vers le haut, la bague 62; cette gorge 70 est délimitée notamment par deux flancs 71 et 72 parallèles entre eux et définis par des génératrices parallèles à l'axe 4, lesquels flancs 71 et 72 sont mutuellement distants d'une largeur de gorge 70 sensiblement égale au diamètre du doigt 80; la gorge 70 relie ainsi la face 63 de la bague 62 à une face 73 de cette bague également cylindrique de révolution autour de l'axe 4 mais qui délimite la bague 62 dans le sens d'un éloignement radial vis-à-vis de cet axe, suivant un plan moyen 74 par rapport auquel les deux flancs 71 et 72 de la gorge 70 présentent une courbure telle qu'une rotation de la tige 32 autour de l'axe 77 par rapport au support 1 provoque, du fait de la rotation concomitante du doigt excentré 79 engagé dans la gorge 70, une rotation de la bague 62 autour de l'axe 4, par rapport au manchon 50, dans un sens ou dans l'autre en fonction du sens de rotation de la tige 32; la forme de la gorge 70 est néanmoins telle que, si l'on libère la tige 32 à la rotation autour de l'axe 77 par rapport au support 1, le ressort 58 provoque automatiquement un mouvement du manchon 50 vers le haut, par rapport au chariot 29, en entraînant la bague 62 et avec elle la tige 32 à la rotation autour des axes respectifs 4 et 77 par rapport au support 1 dans un sens tel que les galets 65 gagnent des zones inférieures des parties 61b associées de la face 61, et un pivotement des porte-lame 23 dans un sens tel que la zone active 25 des lames 22 se déplace en éloignement vis-à-vis de l'axe 4, jusqu'à ce que la butée s'établisse entre la partie 75 des porte-lame 23 et le manchon 76.

La manœuvre de la tige 32 a donc essentiellement pour but de ramener les lames en rapprochement de leur zone active 25 par rapport à l'axe 4, pour définir leur limite de départ dans un mouvement d'éloignement vis-à-vis de cet axe, par une rotation manuelle de cette tige 32, autour de l'axe 77, dans un sens tendant à dégager les galets 65 des zones en creux 61b de la face 61 de la pièce 57, c'est-à-dire à rapprocher ces galets 65, par roulement sur les zones 61b, des zones 61a.

Pour permettre de fixer provisoirement cette limite, et ceci à volonté, la tige 32 porte au-dessus de la paroi 12, c'est-à-dire à l'extérieur du volume 14, un bouton de manœuvre qui en est solidaire à la rotation autour de l'axe 77 par rapport au manchon 35 et à la paroi 12, et qui peut lui-même être immobilisé de façon provisoire, et avec lui la tige 32, à l'encontre d'une telle rotation.

A cet effet, le bouton 180 est monté sur la tige 32 au coulissement relatif parallèlement à l'axe 77, directement et immédiatement au-dessus du manchon 35 à la partie inférieure 81 du bouchon 80, et par l'intermédiaire d'un manchon 82 intégralement solidaire de la tige 32 à la partie supérieure 83 du bouton 180; dans cette zone, ce bouton 180 porte de façon solidaire une goupille 84 traversant radialement, en référence à l'axe 77, une lumière 85 allongée suivant l'axe 77, laquelle traverse de part en part le manchon 82 et la tige 32 pour assurer la solidarisation mutuelle du bouton 180 et de la tige 32 à la rotation autour de l'axe 77 en autorisant un débattement par translation relative parallèlement à cet axe; en outre, la partie inférieure 81 du bouton 180 présente vers le bas, autour du manchon 35, une face annulaire plane 86, de révolution autour de l'axe 77 auquel elle est perpendiculaire, cette face annulaire plane 86 étant munie d'une nervure 87 orientée radialement en référence à l'axe 77 et formant saillie vers le bas, sous cette face 86; entre cette dernière et la paroi 12 du support est intercalée une bague 88 disposée autour du manchon 35 avec possibilité de rotation relative autour de l'axe 77 et de translation relative parallèlement à cet axe, cette bague 88 présentant vers le bas une face annulaire plane 89, perpendiculaire à l'axe 77, en vue de son appui sur la paroi 12; vers le haut, la bague 88 présente une paroi également annulaire plane 90, perpendiculaire à l'axe 77 et portant une rainure 91 orientée radialement en référence à cet axe 77, laquelle rainure présente une forme complémentaire de celle de la nervure 87 de la face 86 du bouton 180 pour autoriser un emboîtement mutuel du bouton 180 et de la bague 88 avec solidarisation mutuelle à la rotation autour de l'axe 77; un ressort de compression 92 est logé dans une chambre 93 que le bouton 180 délimite intérieurement autour de la tige 32, et prend appui d'une part vers le haut contre une face inférieure 94 du manchon 82, plane et annulaire, d'axe 77, fermant la chambre 93 vers le haut, et d'autre part vers le bas contre une face annulaire plane 95, perpendiculaire à l'axe 77, tournée vers le haut, que le bouton 180 présente à sa partie inférieure 81, autour de la tige 32, et ce ressort 92 sollicitant élastiquement le bouton 180 vers le bas tend à maintenir la nervure 87 et la rainure 91 en prise mutuelle et la bague 88 en appui vers le bas, par sa face 89, contre la paroi 12; en tirant le bouton 180 vers le haut, on peut néanmoins dégager la nervure 87 de la rainure 91 et par conséquent désolidariser le bouton 180 et la bague 88 à la rotation relative autour de l'axe 77; la nervure et la lumière 85 sont convenablement dimensionnées à cet effet.

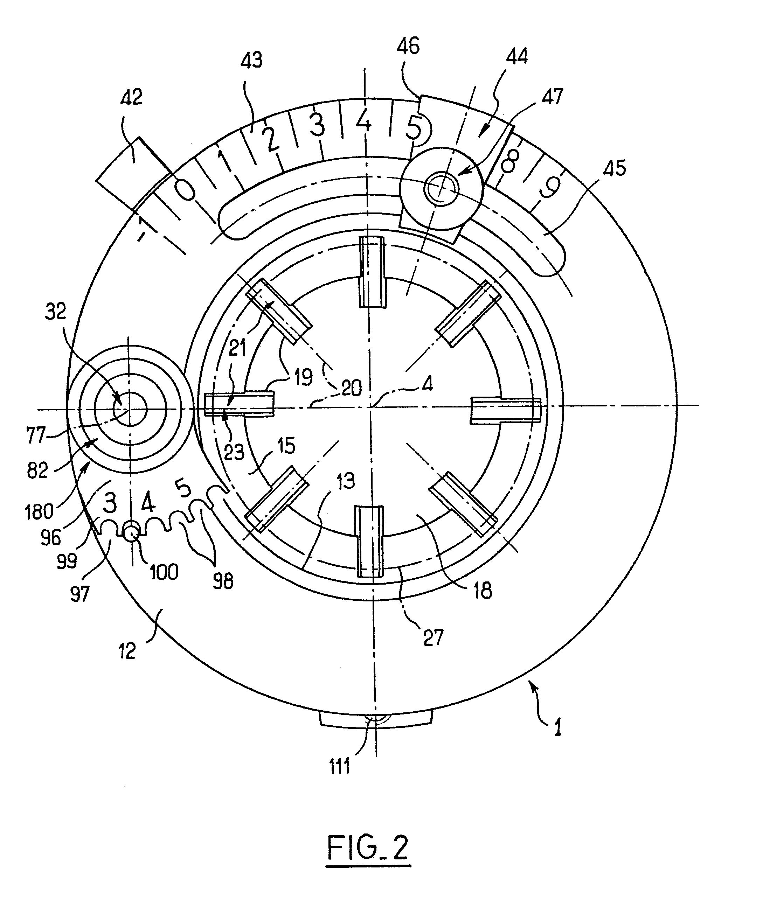

Afin de permettre une immobilisation du bouton 180, et avec lui de la tige 32 dans une position angulaire déterminée, en référence à l'axe 77, par rapport à la paroi 12 du support 1, c'est-à-dire pour immobiliser la bague 62 dans une position angulaire déterminée, en référence à l'axe 4, par rapport au support 1 et ainsi définir une position du manchon 50 à la translation relative, par rapport au chariot 29, parallèlement à l'axe 4, la bague 88 et la paroi 12 portent complémentairement des moyens de solidarisation mutuelle dans des positions relatives indexées; par exemple, comme il ressort plus particulièrement de la figure 2, la bague 88 porte de façon solidaire une plaquette 96, présentant une face inférieure coplanaire avec la face 89, et cette plaquette 96 présente une périphérie 97 radialement extérieure, en référence à l'axe 77, présentant une pluralité de crans 98 assortis d'une graduation 99; ces crans 98 placés suivant un même cylindre virtuel centré sur l'axe 77 peuvent être engagés, au choix, sur un pion 100 qui forme une saillie vers le haut, parallèlement à l'axe 4, au-dessus de la paroi 12 dont il est solidaire; la saillie formée par ce pion 100 au-dessus de la paroi 12 parallèlement à l'axe 4 est inférieure au débattement possible du bouton 180 et avec lui de la bague 88, à la translation parallèlement à l'axe 77, de telle sorte qu'en tirant ensemble le bouton 180 et la bague 88 vers le haut, par rapport au support 1, en comprimant le ressort 92, on puisse dégager du pion 100 un cran 98 initialement engagé sur celui-ci, puis amener à volonté un autre des crans 98 en regard du pion 100 et, en relâchant le bouton 180 et la bague 88, autoriser leur descente à nouveau vers le support 1 sous l'action du ressort 92 et par conséquent l'engagement du cran 98 choisi sur le pion 100, ce qui assure une nouvelle immobilisation de la bague 88, et avec elle du bouton 180, à la rotation autour de l'axe 77 par rapport à la paroi 12 et définit une position du manchon 50 à la translation, parallèlement à l'axe 4, par rapport au chariot 29.

Ainsi peuvent être définies, par engagement des différents crans 98 sur la saillie 100, plusieurs positions des zones actives 25 des lames 22 en écartement par rapport à l'axe 4, avant mouvement de ces lames dans le sens d'un éloignement vis-à-vis de cet axe 4 sous l'action du ressort 58; par exemple, on peut ainsi faire varier de 0,5 mm en 0,5 mm, d'une valeur allant de 3 à 5,5 mm, la distance entre les zones actives 25 de deux lames 22 situées dans deux plans 20 coïncidants, avant éloignement relatif de ces parties actives 25 sous l'action du ressort 58.

Une indexation de la limite des zones actives 25 des lames 22 en rapprochement vis-à-vis de l'axe constitue l'un des réglages préalables à une intervention au moyen de l'appareil; un autre réglage préalable à une telle intervention est celui du coulisseau 44 le long de la lumière 45, pour établir une valeur limite de la saillie que peuvent former les zones actives 25 des lames 22 par rapport à la surface de référence 3; en outre, préalablement à l'intervention, le doigt 42 peut être soit amené dans une position telle que les zones actives 25 des lames 22 soient escamotées par rapport à la surface de référence 3, soit en butée contre le coulisseau 44 de telle sorte que les zones actives 25 des lames forment la saillie ainsi prédéterminée; alors, moyennant un repérage de l'axe optique, on met le support 1 en place sur l'œil 2 en plaçant la surface de référence 3 au contact de celui-ci dans une position telle que l'axe 4 du support coïncide avec l'axe optique 5, en provoquant la pénétration des lames 22 dans la cornée si ces lames sont déjà en saillie; on met alors en route l'aspiration dans la chambre 9 pour assurer la fixation de l'appareil sur l'œil et plaquer la cornée contre la surface de référence, que la cornée épouse étroitement y compris dans la zone 3a; ensuite, dans l'hypothèse où les zones actives 25 des lames 22 étaient initialement escamotées, on provoque leur pénétration dans la cornée, par saillie par rapport à la surface de référence 3, en amenant le doigt 42 progressivement en butée contre le coulisseau 44; on réalise ainsi initialement l'extrémité de chaque incision la plus proche de l'axe 4, avec une profondeur déterminée par la saillie que forme alors la zone active 25 de chaque lame 22 par rapport à la surface de référence 3, et plus précisément par rapport à la zone 3a de cette surface; ensuite, en soulevant le bouton 180, on libère celui-ci vis-à-vis de la bague 88 puis, en laissant tourner librement ce bouton 180 autour de l'axe 77 par rapport au support 1, pour laisser tourner librement la bague 62 autour de l'axe 4 par rapport au chariot 29, on laisse agir librement le ressort 58 qui provoque un mouvement ascendant du manchon 50 par rapport au chariot 29 moyennant roulement des galets 65 sur les zones 61b de la face 61 de la pièce 59, dans un sens correspondant à une descente de la pente de ces zones 61b; on remarquera que, pour autoriser un tel parcours des zones 61b de la face 61 par les galets 65, ce qui signifie un rapprochement de la pièce 57 vis-à-vis de la bague 62, il peut être judicieux d'aménager dans cette dernière des lumières telles que 101, de révolution autour de l'axe 4, en vue de la réception des zones 61a de la face 61 de la pièce 59 lorsque les galets 65 parviennent en bas des zones 61b de la face 61, ce qui correspond au rapprochement maximal de la pièce 59 vis-à-vis de la bague 62; lors de ce mouvement du manchon 50 sous l'action du ressort 58, les différents porte-lames 23 basculent à l'unisson dans un sens correspondant, pour les zones actives 25 de différentes lames 22, à un éloignement conjoint vis-à-vis de l'axe 4, et ce mouvement se poursuit jusqu'à ce que les zones 75 des différents porte-lames 23 viennent en butée contre le manchon 76, ce qui définit la limite de chaque incision la plus éloignée de l'axe 4; entre ces deux limites, l'incision présente une profondeur déterminée par la saillie que forme la zone active 25 de la lame correspondante 22 par rapport à la zone 3a de la surface de référence 3; on remarquera que cette saillie, et avec elle la profondeur d'incision, est fonction d'une part de la position donnée au chariot 29 par action sur le doigt 42, c'est-à-dire du réglage du coulisseau 44, et d'autre part de la géométrie de la zone 3a, que l'on peut avantageusement changer par changement de la lentille 18 qui la définit, de façon à obtenir une profondeur d'incision constante ou, au contraire, une profondeur d'incision variant de façon prédéterminée selon l'éloignement vis-à-vis de l'axe 4; l'obtention d'une profondeur d'incision constante est assurée si, dans chaque plan 20 incluant l'axe 4, la zone 3a se présente sous la forme d'un arc de cercle centré sur l'axe 28 perpendiculaire à ce plan 20; une approximation de cette forme, permettant de conserver une même lentille 18 pour différents réglages possibles de la position du manchon 29 par rapport au support 1 parallèlement à l'axe 4, est néanmoins admissible; lorsque les incisions sont ainsi terminées, on interrompt l'aspiration dans la chambre 9 et on escamote à nouveau les zones actives 25 des lames 22 par rapport à la surface de référence 3, soit en détachant le support 1 de la cornée 2, soit préalablement à un tel détachement, avant démontage, nettoyage, et stérilisation de l'appareil.

Naturellement, on peut apporter de nombreuses variantes à l'appareil qui vient d'être décrit, quant à sa structure concrète comme en ce qui concerne sa géométrie.

On a illustré l'une de ces variantes possibles aux figures 4 et 5, où l'on retrouve respectivement sous les références 201 à 226, 228 à 230, 232, 234 à 237, 250, 252 à 257, 261 à 270, 272, 273, 275 à 296, 305, 311, 341, 380 à d'éventuelles différences géométriques secondaires près, les éléments décrits précédemment sous les références 1 à 26, 28 à 30, 32, 34 à 37, 50, 52 à 57, 61 à 70, 72, 73, 75 à 96, 105, 111, 141, 180; les éléments correspondant aux éléments 27, 71, 74, 97 à 101 du mode de réalisation décrit en référence aux figures 1 à 3 se retrouvent également dans le cas de ce mode de réalisation des figures 4 et 5, mais ne sont pas visibles; pour l'ensemble de ces éléments communs, on se référera à la description des éléments respectivement correspondants du mode de réalisation illustré aux figures 1 à 3.

Par contre, les éléments portant les références 38 à 49, constituant une partie des moyens de déplacement du chariot 29 à la translation par rapport au support 1 parallèlement à l'axe 4 et de limitation de cette translation dans le cas du mode de réalisation illustré aux figures 1 à 3, de même que les éléments 58 à 60 de ce mode de réalisation, ont été remplacés par des moyens différents dans le mode de réalisation des figures 4 et 5.

Le ressort hélicoïdal de compression 58, d'axe 4, a été remplacé par une pluralité de ressorts hélicoïdaux de compression 258 logés dans le volume 214 et présentant des axes respectifs parallèles à l'axe 204, ces axes étant régulièrement répartis angulairement autour de cet axe 204 et les ressorts 258 agissant, de la même façon que le ressort 58, entre l'épaulement 256 de la paroi 211 et l'épaulement 257 du manchon 250.

Les pièces 59 et 60 ont quant à elles été supprimées, le manchon 250 présentant directement vers le haut, la face annulaire 261, présentant des zones planes 261a alternant avec des zones pentues, en creux 261b en vue du roulement des galets 265 de la bague 262; on remarquera que la face 261 présente en outre, dans le cas de ce mode de réalisation, des encoches 261c de passage des galets 267 de roulement de la bague 262 contre la face inférieure 269 du rebord 237 du chariot 229; en effet, dans ce mode de réalisation, les axes 266 et 268 des galets 265 et 267 sont placés coplanairement, dans un même plan perpendiculaire au plan 204, afin de permettre une réduction de l'encombrement de l'appareil dans le sens de l'axe 204.

En outre, dans le mode de réalisation des figures 4 et 5, le guidage du manchon 250 à la translation par rapport au chariot 229, parallèlement à l'axe 204, est assuré non pas par coulissement contre le support 201 comme c'est le cas dans l'exemple des figures 1 à 3, mais par appui glissant d'une portée annulaire 251 du manchon 250, laquelle est cylindrique de révolution autour de l'axe 204 et tournée vers celui-ci, contre la face 264 du chariot 229 contre laquelle glisse également la bague 262.

Quant à eux, les éléments 38 à 49 du mode de réalisation des figures 1 à 3 ont été remplacés de la façon suivante.

Dans ce mode de réalisation illustré aux figures 4 et 5, entre une face annulaire supérieure 238 plane, de révolution autour de l'axe 204, du rebord 237 du chariot 229 et une face inférieure 239, également plane, de révolution autour de l'axe 204, que la paroi 212 présente à l'intérieur du volume 214 est disposée une couronne annulaire 240 d'axe 204, en contact à plat, vers le haut, avec la face 239 par l'intermédiaire d'une garniture de glissement 241; entre la face 230 de la paroi 213, laquelle face est cylindrique de révolution autour de l'axe 204 et tournée dans le sens d'un éloignement vis-à-vis de celui-ci, à l'intérieur du volume 214, et une face 242, également cylindrique de révolution autour de l'axe 204 et tournée vers ce dernier, que la couronne 240 présente est interposé un roulement à billes 243 qui assure un guidage de la couronne 240 à la rotation autour de l'axe 204 par rapport au support 1, une translation relative étant quant à elle empêchée du fait de l'action des ressorts 258 qui, en sollicitant élastiquement le manchon 250 à la translation vers le haut, parallèlement à l'axe 204, par rapport au support 201, sollicitent de la même façon la bague 262 par l'intermédiaire des galets 266, de même que le rebord 237 du chariot 229 par l'intermédiaire des galets 267, le rebord 237 sollicitant lui-même la couronne 240 vers le haut par l'intermédiaire de moyens d'appui mutuel; ces moyens comportent sur le rebord 237 une pluralité de galets 244 montés à rotation sur ce rebord autour d'axes respectifs 245 radiaux par rapport à l'axe 204 et régulièrment répartis angulairement par rapport à celui-ci, et sous la couronne 240 un chemin de roulement 246 annulaire, d'axe 204, présentant des zones 246a coplanaires, de révolution autour de l'axe 204 auquel elles sont perpendiculaires, entre lesquelles s'intercalent des zones en creux 246b pentues en référence à l'axe 204 et par exemple constituées par des portions d'hélice de même pente, d'axe 204; chacune des zones 246b est placée en regard d'un galet 244 si bien que les zones 246b et les galets 244 coopèrent à la façon de cameset de toucheaux came, respectivement, de telle sorte qu'une rotation de la couronne 240 dans un sens 247 autour de l'axe 204 par rapport au support 1 se traduise par un contact de chaque galet 244 avec des zones de plus en plus basses de la partie 246b du chemin de roulement que constitue le bord 246, c'est-à-dire des zones dont le niveau est de plus en plus proche de celui des zones 246a, ce qui se traduit par une descente du chariot 229 parallèlement à l'axe 204 par rapport au support 201; on remarquera que, dans cet exemple, un tel mouvement est facilité par le remplacement de la portée annulaire 31 décrite en référence aux figures 1 à 3 par un roulement à billes 248 intercalé entre la face 230 de la paroi 213 et une face 249, cylindrique de révolution autour de l'axe 204 et tournée vers ce dernier, que présente le rebord 237 du chariot 229.

La pente des zones 246b constituant le chemin de roulement des galets 244 sous la couronne 240, ainsi que la qualité de ce roulement et l'aptitude au glissement de la couronne 240 par rapport à la paroi 212 grâce à la garniture 241 sont choisies telles qu'il y ait réversibilité de mouvement, c'est-à-dire que la sollicitation du rebord 237 vers le haut par les ressorts 258 se traduise par une rotation de la couronne 240 en sens inverse du sens 247, autour de l'axe 204, par rapport au support 201, au cours de laquelle les galets 244 gagnent les zones les plus profondes, c'est-à-dire les plus hautes de leur chemin de roulement 246b; ainsi, les ressorts 258 agissent dans le sens d'un escamotage des zones actives 225 des lames 222, par rapport à la surface de référence 203; par ailleurs, comme dans le cas du mode de réalisation illustré aux figures 1 à 3, les ressorts 258 tendent à faire tourner la bague 262 autour de l'axe 204 par rapport au chariot 229, dans le sens 227 qui est opposé au sens 247, par roulement de chacun des galets 265 vers les zones les plus basses de leur chemin de roulement 261b, ce qui correspond à placer les parties actives 225 des lames 222 au plus près de l'axe 204 si un verrouillage du bouton 280 de manœuvre de la tige 232 ne s'y oppose pas, comme il a été dit plus haut; on remarquera que, dans l'exemple illustré aux figures 4 et 5, la réversibilité de la transmission de mouvement entre la tige 232 et la bague 262 est améliorée par un roulement à billes 271 par lequel le doigt 279 entre en contact avec les flancs tels que 272 de la gorge 270 de la bague 262.

Pour permettre une manœuvre de la couronne 240 à la rotation autour de l'axe 204 par rapport au support 201, à volonté, et une immobilisation à volonté à l'encontre d'une rotation relative, la couronne 240 est percée, suivant un axe 274 parallèle à l'axe 204, d'un alésage taraudé 298 dans lequel est vissée une tige 299 d'axe 274, laquelle traverse la paroi 212 via une lumière 300 aménagée dans celle-ci avec une forme de révolution autour de l'axe 204; la tige 299 porte au-dessus de la paroi 112 un bouton de manœuvre 301 qui permet de visser ou de dévisser la tige 299 vis-à-vis de l'alésage 298 et ainsi, respectivement, d'assurer une immobilisation de la couronne 240 vis-à-vis de la paroi 212 par serrage de cette dernière entre la couronne 240 et le bouton 301, ou au contraire de libérer la couronne 240 vis-à-vis d'une rotation par rapport à la paroi 212, autour de l'axe 204, ce qui permet d'amener, par le jeu des galets 244 et de leurs chemins de roulement 246b, le chariot 229 dans toute position voulue, à la translation parallèlement à l'axe 204, par rapport au support 201 afin d'escamoter les parties actives 225 des lames 222 par rapport à la surface de référence 203, ou au contraire de les placer en saillie par rapport à cette dernière, d'une valeur déterminée; avantageusement, une graduation analogue à la graduation 43 visible à la figure 2 est portée sur la paroi 212 pour offrir un repérage de la position angulaire, en référence à l'axe 204, de la couronne 240 par rapport au support 201.

On remarquera que la différence de structure des moyens de manœuvre du manchon 250 qui vient d'être décrite n'affecte en rien le fonctionnement de l'appareil, si bien que la description qui a été faite de ce fonctionnement en référence aux figures 1 à 3 peut être reprise à propos des figures 4 et 5.

Incidemment, on remarquera que la couronne 240, dont le dimensionnement radial, en référence à l'axe 204, est aussi grand que le permet le volume 214, est percée de part en part, parallèlement à l'axe 204, d'une lumière 302 axée sur cet axe 204, pour autoriser le passage de la tige 232 et d'un prolongement annulaire du manchon 235 de celle-ci jusqu'à sa traversée du rebord 237 via un alésage 236 de celui-ci.

Outre les différences qui viennent d'être décrites, communiquant à l'appareil illustré aux figures 4 et 5 les mêmes possibilités que dans le cas de l'appareil illustré aux figures 1 à 3, l'appareil illustré aux figures 4 et 5 présente des caractéristiques lui offrant une possibilité supplémentaire; en effet, dans le cas de cet appareil, la paroi 215 est limitée dans le sens de la hauteur de telle sorte qu'il ne soit pas nécessaire d'y aménager les fentes 219, aménagées dans ce cas exclusivement dans la lentille 218, et cette dernière est solidarisée de façon amovible avec la paroi 215 par des moyens assurant une étanchéité, si bien que l'ensemble formé par les volumes 214 et 216 est délimité de façon étanche par le support 201, et ouvert exclusivement vers la surface de référence 203, par les fentes 219 de la lentille 218; un raccord 302 en tout point comparable au raccord 311 permet de raccorder ce volume 214–216 à une source d'aspiration, ce qui améliore le plaquage de la cornée contre la lentille et neutralise la résistance de la cornée à la pénétration des lames, aboutissant ainsi à une reproducibilité de la profondeur d'incision.

Par exemple, à cet effet, la lentille 218 présente, dans une zone supérieure, un épaulement annulaire 303, plan, de révolution autour de l'axe 204, par lequel elle s'appuie sur un épaulement annulaire 304, de révolution autour de l'axe 204 et quant à lui tourné vers le haut, que la paroi 215 présente en partie inférieure; la paroi 213 présente quant à elle, vers l'axe 204, un taraudage 335 permettant d'y visser une bague 306, de révolution autour de l'axe 204, présentant un filetage complémentaire 307; vers le bas, la bague 306 présente un épaulement annulaire 308 de révolution autour de l'axe 204, lequel épaulement annulaire 308 permet d'appuyer la lentille 218 vers le bas sur l'épaulement 304, en s'appliquant sur la lentille par l'intermédiaire d'un joint torique 309, de révolution autour de l'axe 204, prenant appui vers le bas à la fois sur la lentille et sur une zone de la paroi 215 immédiatement adjacente; un positionnement précis de la lentille 218 par rapport au support 201, en référence à l'axe 204, est assuré par un pion 310 solidaire de la lentille 218 et formant saillie en dessous de l'épaulement 303 de celle-ci pour pénétrer dans une encoche complémentaire 321 de l'épaulement 304 de la paroi 215.

Naturellement, un montage analogue pourrait être adopté sur un appareil conforme par ailleurs aux figures 1 à 3.

De même, on pourrait prévoir sur un tel appareil, comme il est illustré à la figure 4, un anneau 312 encastrable à l'embouchure 210 de la chambre d'aspiration 209, sans obturer celle-ci, cet anneau 312 présentant vers le bas une indentation 313 d'ancrage sur la cornée.

De même, on pourrait adopter sur l'appareil illustré aux figures 1 à 3 le montage des lames 222 dans les bras 224 des porte-lame 223 qui est illustré à la figure 4 et à la figure 5, à savoir un montage selon lequel le bras 224, définissant comme il a été dit plus haut un logement 314 de réception de la lame 222, porte de façon solidaire deux goupilles traversant ce logement 314 de part en part parallèlement à l'axe 228 d'articulation sur le chariot 229; dans l'exemple illustré, l'une 315 de ces goupilles, relativement supérieure, définit également le tourillon d'articulation du porte-lames autour de l'axe 228 sur le chariot 229 alors que l'autre goupille 316, relativement inférieure, est située à proximité de la limite inférieure du bras 224; à l'intérieur du logement 314 est disposé un ressort à lame 317 présentant deux encoches 318 et 319 de réception respectivement de l'une et l'autre des goupilles 315 et 316, ce ressort 317 présentant, lorsqu'il est vu dans un plan 320 pas-

sant par l'axe 228 et par un axe 321 de la goupille 316, une courbure telle qu'il s'appuie à proximité immédiate des encoches 318 et 319 d'un côté du logement 314, et dans une zone intermédiaire entre les deux encoches 318 et 319 de l'autre côté du logement 314.

Complémentairement, la lame 222 présente, dans une zone 322 s'engageant dans le logement 314, une fente 324 ouverte à l'opposé de la partie active 225 et présentant vers celle-ci un fond fermé, laquelle fente est propre à épouser extérieurement les deux goupilles 315 et 316 en s'appuyant en outre vers le haut, par son fond, contre la goupille 316; du fait de l'incurvation du ressort 317, ce dernier plaque la partie 322 de la lame 222 du côté du logement 314 sur lequel tand à s'appuyer sa zone intermédiaire entre les encoches 318 et 319, pour assurer une retenue élastique de la lame 222 dans le bras 224 dans une position telle qu'un plan moyen 323 de la lame 222 inclue l'axe 204 du support 201, et coïncide avec le plan 220 de la fente 219 associée.

Un tel montage offre toute sécurité, tout en autorisant une extraction et une mise en place rapides de la lame 222 par rapport au porte-lame 223; on prévoit en outre à cet effet, avantageusement, un trou 325 traversant la lame 222 de part en part à la partie supérieure de sa partie active 225, c'est-à-dire à proximité immédiate de sa partie 322 engagée dans le bras 224 du porte-lame 223.

On remarquera qu'il n'est pas possible d'intercaler entre la goupille 316 et le fond de la fente 324 une cale d'épaisseur pour augmenter la saillie que la zone active 225 d'une lame 222 forme sous le bras 224 du porte-lame associé 223 et, en associant à des lames identiques de telles cales d'épaisseur différente, de pratiquer simultanément des incisions de profondeur différente en vue de corrections de myopies avec astigmatisme; de même, il est possible d'utiliser simultanément des lames de géométrie différente.

Ces lames pourront être réalisées en acier, éventuellement garni de diamant dans leur zone active, ces exemples n'étant nullement limitatifs.

## Revendications

1. Appareil de kératotomie radiaire, comprenant un support (1, 201) présentant:
   - un axe (4, 204) du support (1, 201),
   - une surface de référence (3, 203) concave, sécante de l'axe (4, 204) du support (1, 201) et propre à épouser la cornée (2a, 202a) d'un œil dans une position dans laquelle l'axe (4, 204) du support (1, 201) coïncide avec l'axe optique (5, 205) de l'œil (2, 202).,
   - une pluralité de fentes (19, 219) dont chacune est disposée selon un plan respectif (20, 220) incluant l'axe (4, 204) du support (1, 201) et débouche dans une zone (3a, 203a) de la surface de référence (3, 203), chaque fente (19, 219) autorisant le passage d'une lame (22, 222) de telle sorte que ladite lame (22, 222) présente une partie active (25, 225) formant une saillie déterminée par rapport à la surface de référence (3, 203) et puisse évoluer suivant ledit plan (20, 220) de la fente (19, 219) entre deux limites déterminées, respectivement de rapprochement et d'éloignement de la partie active (25, 225) de la lame (22, 222) par rapport à l'axe (4, 204) du support (1, 201),

caractérisé en ce que le support (1, 201) porte une pluralité de lames (20, 220) dont chacune est associée à une fente respective (19, 219), et des moyens (50, 58, 61, 62, 65, 67, 69, 70, 80, 32, 180, 250, 258, 261, 262, 265, 267, 269, 270, 280, 232, 380), disposés en retrait par rapport à la surface de référence (3, 203) pour provoquer un déplacement simultané des lames (22, 222) par rapport au support (1, 201), dans les fentes (19, 219) respectivement associées et selon ledit plan respectif (20, 220) de celles-ci, d'une même première desdites deux limites déterminées respectives à la deuxième de ces deux limites déterminées respectives alors qu'une partie active (25, 225) de chaque lame (22, 222) forme une saillie respective déterminée par rapport à la surface de référence (3, 203).

2. Appareil selon la revendication 1, caractérisé en ce qu'il comporte des moyens (29, 38, 39, 40, 42, 229, 240, 244, 246, 258, 301) d'escamotage réversible de la partie active (25, 225) des lames (22, 222) par rapport à la surface de référence (3, 203).

3. Appareil selon l'une quelconque des revendications 1 et 2, caractérisé en ce que les lames (22, 222) sont amovibles et interchangeables.

4. Appareil selon l'une quelconque des revendications 1 à 3, caractérisé en ce que la surface de référence (2, 202) est amovible et interchangeable au moins quant à une zone annulaire (3a, 203a) située autour de l'axe (4, 204) du support (1, 201) et dans laquelle débouchent les fentes (19, 219).

5. Appareil selon l'une quelconque des revendications 1 à 4, caractérisé en ce que la surface de référence (3, 203) comporte une face d'une lentille (18, 218) formant partie intégrante du support (1, 201) et présentant lesdites fentes (19, 219).

6. Appareil selon l'une quelconque des revendications 1 à 5, caractérisé en ce qu'il comporte des moyens (9, 10, 111, 209, 210, 311, 216, 302) de plaquage pneumatique de la cornée (2a, 202a) contre la surface de référence (3, 203).

7. Appareil selon la revendication 6, caractérisé en ce que le support (201) délimite de façon étanche, autour des lames (222), un volume (216) ouvert exclusivement vers la surface de référence (3, 203) notamment via lesdites fentes (19, 219), et en ce qu'il comporte des moyens (302) de raccordement dudit volume à une source d'aspiration.

8. Appareil selon l'une quelconque des revendications 6 et 7, caractérisé en ce que la surface de référence (3, 203) présente une convexité limitée à une zone annulaire (3a, 203a) située autour de l'axe (4, 204) du support (1, 201) et dans laquelle débouchent les fentes (19, 219).

9. Appareil selon l'une quelconque des revendications 1 à 8, caractérisé en ce qu'il comporte des moyens (29, 38, 39, 40, 42, 44, 229, 240, 244, 246, 258, 301) pour régler ladite saillie par rapport à la surface de référence (3, 203).

10. Appareil selon l'une quelconque des revendications 1 à 9, caractérisé en ce qu'il comporte des moyens (88, 96, 98, 100, 288, 296) pour régler la position de ladite première limite en écartement par rapport à l'axe (4, 204) du support (1, 201).

11. Appareil selon l'une quelconque des revendications 1 à 10, caractérisé en ce qu'il comporte des moyens (76, 276) pour régler la position de ladite deuxième limite en écartement par rapport à l'axe du support.

12. Appareil selon l'une quelconque des revendications 1 à 11, caractérisé en ce que lesdites première et deuxième limites sont respectivement la limite en rapprochement et la limite en éloignement de la partie active (25, 225) de chaque lame (22, 222) par rapport à l'axe (4, 204) du support (1, 201).

13. Appareil de kératotomie radiaire, comprenant un support (1, 201) présentant:

– un axe (4, 204) du support (1, 201),

– une surface de référence (3, 203) concave, sécante de l'axe (4, 204) du support (1, 201) et propre à épouser la cornée (2a, 202a) d'un œil dans une position dans laquelle l'axe (4, 204) du support (1, 201) coïncide avec l'axe optique (5, 205) de l'œil (2, 202),

– une pluralité de fentes (19, 219) dont chacune est disposée selon un plan respectif (20, 220) incluant l'axe (3a, 203a) de la surface de référence (3, 203), chaque fente (19, 219) autorisant le passage d'une lame (22, 222) de telle sorte que ladite lame (22, 222) présente une partie active (25, 225) formant une saillie déterminée par rapport à la surface de référence (3, 203) et puisse évoluer suivant ledit plan (20, 220) de la fente (19, 219) entre deux limites déterminées, respectivement de rapprochement et d'éloignement de la partie active (25, 225) de la lame (22, 222) par rapport à l'axe (4, 204) du support (1, 201), caractérisé en ce qu'il comporte:

– un chariot annulaire (29, 229) disposé autour de l'axe (4, 204) du support (1, 201), en retrait par rapport à la surface de référence (3, 203),

– des moyens (30, 31, 32, 232, 248) de guidage du chariot (29, 229) à la translation par rapport au support (1, 201) parallèlement à l'axe (4, 204) du support (1, 201),

– des moyens (42, 44, 48, 301) définissant deux positions limites stables, déterminées, du chariot (29, 229) par rapport à la surface de référence (3, 203) du support (1, 201) lors de ladite translation du chariot (29, 229) par rapport au support (1, 201),

– des moyens (38, 39, 40, 42, 240, 244, 246, 258, 301) pour manœuvrer à volonté le chariot (29, 229) à la translation par rapport au support (1, 201) entre lesdites positions limites,

– une pluralité de porte-lame (23, 223) dont chacun est associé à une fente respective (19, 219) et monté au pivotement sur le chariot (29, 229) autour d'un axe respectif (28, 228) perpendiculaire audit plan (20, 220) de la fente associée (19, 219),

– une pluralité de lames (22, 222) dont chacune est solidaire d'un porte-lame (23, 223) respectif, ladite lame (22, 222) étant disposée selon ledit plan (20, 220) de la fente (19, 219) associée et présentant au travers de la fente associée (19, 219) une partie active (25, 225) au voisinage de la surface de référence (3, 203) ladite partie active (25, 225) étant escamotée par rapport à la surface de référence (3, 203) dans une première desdites positions limites du chariot (29, 229) par rapport à la surface de référence (3, 203) et formant une saillie par rapport à la surface de référence (3, 203) dans la deuxième de ces deux positions limites,

– des moyens (24, 224, 314 à 319, 324) de solidarisation de chacune des lames (22, 222) avec le porte-lame associé (23, 223),

– des moyens (88, 96, 98, 100, 76, 288, 296, 276) définissant deux positions limites stables, déterminées, de chaque porte-lame (23, 223) au pivotement par rapport au chariot (29, 229), définissant elles-mêmes lesdites deux limites déterminées, respectivement de rapprochement et d'éloignement de la partie active (25, 225) de la lame (22, 222) correspondante par rapport à l'axe (4, 204) du support (1, 201),

– des moyens (50, 58, 61, 62, 65, 67, 69, 70, 80, 32, 180, 250, 258, 261, 262, 265, 267, 269, 270, 280, 232, 380) pour manœuvrer à volonté l'ensemble des porte-lame (23, 223) au pivotement par rapport au chariot (29, 229), autour desdits axes de pivotement (28, 228), de telle sorte que les parties actives (25, 225) des lames (22, 222) se déplacent à l'unisson d'une première desdites limites, respectivement de rapprochement et d'éloignement par rapport à l'axe (4, 204) du support (1, 201), à la deuxième de ces deux limites.

14. Appareil selon la revendication 13, caractérisé en ce que les lames (22, 222) et porte-lame (23, 223) présentent des moyens de solidarisation mutuelle amovible (314 à 319, 324).

15. Appareil selon l'une quelconque des revendications 13 et 14, caractérisé en ce que la surface de référence (2, 202) est amovible et interchangeable au moins quant à une zone annulaire (3a, 203a) située autour de l'axe (4, 204) du support (1, 201) et dans laquelle débouchent les fentes (19, 219).

16. Appareil selon l'une quelconque des revendications 13 à 15, caractérisé en ce que la surface de référence (3, 203) comporte une face d'une lentille (18, 218) formant partie intégrante du support (1, 201) et présentant lesdites fentes (19, 219).

17. Appareil selon les revendications 15 et 16 en combinaison, caractérisé en ce que la lentille (18, 218) est amovible.

18. Appareil selon l'une quelconque des revendications 13 à 17, caractérisé en ce que la face de référence (3, 203) présente, en creux, un volume d'aspiration (9, 209, 216) et en ce que le support comporte des moyens de raccordement (111, 311, 302) dudit volume d'aspiration (9, 209, 216) à une source de vide.

19. Appareil selon la revendication 18, caractérisé en ce que le support (201) délimite de façon étanche, autour des lames (222), un volume (216) ouvert exclusivement vers la surface de référence (3, 203) notamment via lesdites fentes (19, 219), et en ce qu'il comporte des moyens (302) de raccordement dudit volume à une source d'aspiration.

20. Appareil selon l'une quelconque des revendications 18 et 19, caractérisé en ce que la surface de référence (3, 203) présente une convexité limitée à une zone annulaire (3a, 203a) située autour de l'axe (4, 204) du support (1, 201) et dans laquelle débouchent les fentes (19, 219), ladite zone annulaire (3a, 203a) présentant une forme correspondant approximativement à une enveloppe d'arcs dont chacun est situé dans ledit plan (20, 220) d'une fente (19, 219) respective et axé sur l'axe (28, 228) de pivotement du porte-lame (23, 223) associé par rapport au chariot (29, 229).

21. Appareil selon l'une quelconque des revendications 13 à 20, caractérisé en ce que la partie active (25, 225) de chaque lame (22, 222) est disposée selon ledit plan (20, 220) de la fente (19, 219) associée, et en ce que les moyens (30, 31, 32, 232, 243) de guidage du chariot (29, 229) à la translation par rapport au support (1, 201) comportent des moyens (32, 36, 232, 236) empêchant toute rotation du chariot (29, 229) par rapport au support (1, 201) autour de l'axe (4, 204) du support (1, 201).

22. Appareil selon l'une quelconque des revendications 13 à 20, caractérisé en ce que la partie active (25, 225) de chaque lame (22, 222) est disposée selon un plan incliné d'un angle déterminé par rapport audit plan (20, 220) de la fente associée (19, 219), cet angle étant identique pour toutes les lames (22, 222), et en ce que les moyens (30, 31, 32, 232, 243) de guidage du chariot (29, 229) à la translation par rapport au support (1, 201) comportent une liaison mutuelle du chariot (29, 229) et du support (1, 201) par une surface de came hélicoïdale axée sur l'axe (4, 204) du support (1, 201) et présentant un angle d'hélice compatible avec ledit angle déterminé pour que, lors de ladite translation du chariot (29, 229) par rapport au support (1, 201), ledit plan de la partie active (25, 225) de chaque lame (22, 222) reste fixe par rapport au support (1, 201).

23. Appareil selon l'une quelconque des revendications 21 et 22, caractérisé en ce que les moyens (38, 39, 40, 42) pour manœuvrer le chariot (29) à la translation par rapport au support (1) comportent une couronne (40) portée par le support (1) avec possibilité de rotation relative autour de l'axe (4) du support (1) sans possibilité de translation relative parallèlement à cet axe (4), des filetages complémentaires (38, 39), en prise mutuelle, aménagés respectivement sur le chariot (29) et sur ladite couronne (40), et des moyens (42) pour manœuvrer à volonté ladite couronne (40) à la rotation par rapport au support (1), autour de l'axe (4) du support (1).

24. Appareil selon l'une quelconque des revendications 21 et 22, caractérisé en ce que les moyens (240, 244, 246, 258, 301) pour manœuvrer le chariot (229) à la translation par rapport au support (201) comportent une couronne (240) portée par le support (201) avec possibilité de rotation relative autour de l'axe (204) du support (201) sans possibilité de translation relative parallèlement à cet axe (204), ladite couronne (240) et le chariot (229) étant en prise mutuelle par l'intermédiaire d'au moins un chemin de came (246) porté par l'un (240) d'eux et pentu (246b) en référence à l'axe (204) du support (201) et d'au moins un toucheau de came (244) porté par l'autre (229) d'entre eux en regard du chemin de came (240), des moyens (258) pour assurer un contact mutuel du toucheau de came (244) et du chemin de came (246), et des moyens (301) pour manœuvrer à volonté ladite couronne (240) à la rotation par rapport au support (201), autour de l'axe (204) du support (201).

25. Appareil selon l'une quelconque des revendications 23 et 24, caractérisé en ce que les moyens (42, 44, 48, 301) définissant deux position limites stables du chariot (29, 229) par rapport à la surface de référence (3, 203) du support (1, 201) comportent des moyens de butée pour imposer à la rotation de la couronne (40, 240) par rapport au support (1, 201), autour de l'axe (4, 204) du support (1, 201), des limites de rotation correspondant auxdites première et deuxième positions limites du chariot (29, 229) par rapport à la surface de référence (3, 203).

26. Appareil selon l'une quelconque des revendications 13 à 25, caractérisé en ce qu'il comporte des moyens (29, 38, 39, 40, 42, 44, 229, 240, 244, 246, 258, 301) pour régler ladite deuxième position limite du chariot (29, 229) par rapport à la surface de référence (3, 203).

27. Appareil selon les revendications 25 et 26 en combinaison, caractérisé en ce qu'il comporte des moyens (44, 301) pour régler au moins celle des limites de rotation de la couronne (40, 240) par rapport au support (1, 201) qui correspond à la deuxième position limite du chariot (29, 229) par rapport à la surface de référence (3, 203).

28. Appareil selon l'une quelconque des revendications 13 à 27, caractérisé en ce qu'il comporte des moyens (88, 96, 100, 228, 296) pour régler la position de ladite première limite en écartement par rapport à l'axe (4, 204) du support (1, 201).

29. Appareil selon l'une quelconque des revendications 13 à 28, caractérisé en ce qu'il comporte des moyens (76, 276) pour régler la position de ladite deuxième limite en écartement par rapport à l'axe du support.

30. Appareil selon l'une quelconque des revendications 13 à 29, caractérisé en ce que les moyens (50, 58, 61, 62, 65, 67, 69, 70, 80, 32, 180, 250, 258, 261, 262, 265, 267, 269, 270, 280, 232, 380) pour manœuvrer l'ensemble des porte-lame (23, 223) au pivotement par rapport au chariot (29, 229) comportent:

– un manchon annulaire (50, 250) coaxial au chariot (29, 229),

– des moyens (51, 52, 54, 23, 251, 264, 254, 223) de guidage du manchon (50, 250) à la translation par rapport au chariot (29, 229) parallèlement à l'axe (4, 204) du support (1, 201) sans possibilité de rotation relative autour de l'axe (4, 204) du support (1, 201),

– des moyens (54, 55, 254, 255) de liaison cinématique entre le manchon (50, 250) et chacun des porte-lame (23, 223), associant à une translation du manchon (50, 250) par rapport au chariot (29, 229) dans l'un ou l'autre de deux sens opposés parallèles à l'axe (4, 204) du support (1, 201) un pivotement simultané de l'ensemble des porte-lame (23, 223) par rapport au chariot (29, 229) de telle sorte que les parties actives (25, 225) des lames (22, 222) se déplacent respectivement en rapprochement ou en éloignement par rapport à l'axe (4, 204) du support (1, 201),

– des moyens (58, 61, 62, 65, 67, 69, 70, 80, 32, 180, 258, 261, 262, 265, 267, 269, 270, 280, 232, 380) pour manœuvrer à volonté le manchon (50, 250) à la translation par rapport au chariot (29, 229) dans l'un et l'autre desdits deux sens opposés.

31. Appareil selon la revendication 30, caractérisé en ce que les moyens (58, 61, 62, 65, 67, 69, 70, 80, 32, 180, 258, 261, 262, 265, 267, 269, 270, 280, 232, 380) pour manœuvrer le manchon (50, 250) à la translation par rapport au chariot (29, 229) comportent:

– des moyens (58, 258) de sollicitation élastique du manchon (50, 250) à la translation dans un premier desdits deux sens opposés par rapport au chariot (29, 229),

– des moyens réversibles (61, 62, 65, 67, 69, 70, 80, 32, 180, 261, 262, 265, 267, 269, 270, 280, 232, 380) de manœuvre du manchon (50, 250) à la translation par rapport au chariot (29, 229) dans le deuxième desdits deux sens opposés,

– des moyens (88, 96, 98, 100, 288, 296) d'immobilisation provisoire du manchon (50, 250) à l'encontre d'une translation sous l'action des moyens de sollicitation élastique (58, 258) par rapport au chariot (29, 229), pour définir ladite première desdites limites respectivement de rapprochement et d'éloignement actives (25, 225) des lames (22, 222) par rapport à l'axe (4, 204) du support (1, 201),

– des moyens (76, 276) pour définir ladite deuxième desdites limites respectivement de rapprochement et d'éloignement des parties actives (25, 225) des lames (22, 222) par rapport à l'axe (4, 204) du support (1, 201).

32. Appareil selon la revendication 31, caractérisé en ce que les moyens réversibles (61, 62, 65, 67, 69, 70, 80, 32, 180, 261, 262, 265, 267, 269, 270, 280, 232, 380) de manœuvre du manchon (50, 250) à la translation par rapport au chariot (29, 229) dans ledit deuxième sens comportent une bague (62, 262) portée par le chariot (29, 229) avec possibilité de rotation relative autour de l'axe (4, 204) du support (1, 201) sans possibilité de translation relative parallèlement à cet axe (4, 204); ladite bague (62, 262) et le manchon (50, 250) étant en prise mutuelle par l'intermédiaire d'au moins un chemin de came (61, 261) porté par l'un (50, 250) d'eux et pentu (61b, 261b) en référence à l'axe (4, 204) du support (1, 201) et d'au moins un toucheau de came (65, 265) porté par l'autre (62, 262) d'entre eux en regard du chemin de came (61, 261), des moyens (58, 258) pour assurer un contact mutuel du toucheau de came (65, 265) et du chemin de came (61, 261), et des moyens (70, 80, 32, 180, 270, 280, 232, 380) pour manœuvrer à volonté ladite bague (62, 262) à la rotation par rapport au chariot (29, 229), autour de l'axe (4, 204) du support (1, 201), et en ce que les moyens (88, 96, 98, 100, 288, 298) d'immobilisation provisoire du manchon (50, 250) à l'encontre d'une translation sous l'action des moyens (58, 258) de sollicitation élastique comportent des moyens (88, 96, 98, 100, 288, 296) d'immobilisation provisoire de ladite bague (62, 262) à l'encontre d'une rotation par rapport au chariot (29, 229) autour de l'axe (4, 204) du support (1, 201).

33. Appareil selon la revendication 32, caractérisé en ce que les moyens (88, 96, 98, 100, 288, 296) d'immobilisation provisoire de ladite bague (62, 262) à l'encontre d'une rotation par rapport au chariot (29, 229) autour de l'axe (4, 204) du support (1, 201) comportent des moyens (96, 98, 100) d'indexation d'une pluralité des positions angulaires relatives d'immobilisation provisoire de ladite bague (62, 262) par rapport au chariot (29, 229), en référence à l'axe (4, 204) du support (1, 201).

34. Appareil selon l'une quelconque des revendications 31 à 33, caractérisé en ce que les moyens (76, 276) pour définir ladite deuxième desdites limites comportent une butée annulaire (76, 276), solidaire du support (1, 201), pour les porte-lame (23, 223).

35. Appareil selon la revendication 34, caractérisé en ce que ladite butée annulaire (76, 276) et le support (1, 201) présentent des moyens de solidarisation mutuelle amovible.

36. Appareil selon l'une quelconque des revendications 30 à 35, caractérisé en ce que lesdits moyens (54, 55, 254, 255) de liaison cinématique entre le manchon (50, 250) et chacun des porte-lame (23, 223) associent à une translation du manchon (50, 250) dans le premier desdits deux sens opposés, par rapport au chariot (29, 229), un pivotement simultané de l'ensemble des porte-lame (23, 223) par rapport au chariot (29, 229) de telle sorte que les parties actives (25, 225) des lames (22, 222) se déplacent de ladite limite de rapprochement par rapport à l'axe (4, 204) du support (1, 201), constituant ladite première limite, à ladite limite en éloignement par rapport à l'axe (4, 204) du support (1, 201), constituant ladite deuxième limite.

## Claims

1. Radial keratotomy apparatus comprising a support (1, 201) having:–

– an axis (4, 204) of the support (1, 201),

– a concave reference surface (3, 203) secant to the axis (4, 204) of the support (1, 201) and adapted to fit the cornea (2a, 202a) of an eye in a position in which the axis (4, 204) of the support (1, 201) coincides with the optical axis (5, 205) of the eye (2, 202),

– a plurality of slots (19, 219) of which each is arranged with a respective plane (20, 220) including the axis (4, 204) of the support (1, 201) and opens into a zone (3a, 203a) of the reference surface (3, 203), each slot (19, 219) permitting the passage of a blade (22, 222) in such a way that said blade (2, 222) has an active part (25, 225) forming a projection determined with respect to the reference surface (3, 203) and can move in the plane (20, 220) of the slot (19, 219) between two determined limits, respectively of approach and extension of the active part (25, 225) of the blade (22, 222) with respect to the axis (4, 204) of the support (1, 201),

characterised in that the support (1, 201) has a plurality of blades (20, 220) of which each is associated with a respective slot (19, 219), and means (50, 58, 61, 62, 65, 67, 69, 70, 80, 32, 180, 250, 258, 261, 262, 265, 267, 269, 270, 280, 232, 380), arranged behind the reference surface (3, 203) for bringing about simultaneous displacement of the blades (22, 222) with respect to the support (1, 201), in the respective associated slots (19, 219) and in their respective planes (20, 220), from a same first of the two said respective determined limits to the second of these two respective determined limits and while an active part (25, 225) of each blade (22, 222) forms a respective determined projection with respect to the reference surface (3, 203).

2. Apparatus according to claim 1, characterised in that it comprises means (29, 38, 39, 40, 42, 229, 240, 244, 246, 258, 301) for reversible withdrawal of the active part (25, 225) of the blades (22, 222) with respect to the reference surface (3, 203).

3. Apparatus according to either claim 1 or claim 2, characterised in that the blades (22, 222) are removable and interchangeable.

4. Apparatus according to any one of claims 1 to 3, characterised in that the reference surface (3, 203) is removable and interchangeable at least as regards an annular zone (3a, 203a) situated around the axis (4, 204) of the support (1, 201) and into which the slots (19, 219) open.

5. Apparatus according to any one of claims 1 to 4, characterised in that the reference surface (3, 203) comprises a face of a lens (18, 218) forming an integral part of the support (1, 201) and having the said slots (19, 219).

6. Apparatus according to any one of claims 1 to 5, characterised in that it comprises means (9, 10, 111, 209, 311, 216, 302) for pneumatic pressing of the cornea (2a, 202a) against the reference surface (3, 203).

7. Apparatus according to claim 6, characterised in that the support (201) limits in a fluid tight manner, around the blades (222), a volume (216) open only towards the reference surface (3, 203) particularly via the said slots (19, 219), and in that it comprises means (302) for connecting the said volume to a source of vacuum.

8. Apparatus according to either claim 6 or claim 7, characterised in that the reference surface (3, 203) has a convexity limited to an annular zone (3a, 203a) situated about the axis (4, 204) of the support (1, 201) and in which the slots (19, 219) open.

9. Apparatus according to any one of claims 1 to 8, characterised in that it comprises means (29, 38, 39, 40, 42, 44, 229, 240, 244, 246, 258, 301) for adjusting the said projection with respect to the reference surface (3, 203).

10. Apparatus according to any one of claims 1 to 9, characterised in that it comprises means (88, 96, 98, 100, 288, 296) for adjusting the position of the said first limit spacedly with respect to the axis (4, 204) of the support (1, 201).

11. Apparatus according to any one of claims 1 to 10, characterised in that it comprises means (76, 276) for adjusting the position of the said second limit spacedly with respect to the axis of the support.

12. Apparatus according to any one of claims 1 to 11, characterised in that the said first and second limits are respectively the limit of approach and the limit of extension of the active part (25, 225) of each blade (22, 222) with respect to the axis (4, 204) of the support (1, 201).

13. Radial keratotomy apparatus comprising a support (1, 201) having:

— an axis (4, 204) of the support (1, 201),

— a concave reference surface (3, 203) secant to the axis (4, 204) of the support (1, 201) and adapted to fit the cornea (2a, 202a) of an eye in a position in which the axis (4, 204) of the support (1, 201) coincides with the optical axis (5, 205) of the eye (2, 202),

— a plurality of slots (19, 219) of which each is arranged with a respective plane (20, 220) including the axis (4, 204) of the support (1, 201) and opens into a zone (3a, 203a) of the reference surface (3, 203), each slot (19, 219) permitting the passage of a blade (22, 222) in such a way that said blade (22, 222) has an active part (25, 225) forming a projection determined with respect to the reference surface (3, 203) and can move in the plane (20, 220) of the slot (19, 219) between two determined limits, respectively of approach and extension of the active part (25, 225) of the blade (22, 222) with respect to the axis (4, 204) of the support (1, 201), characterised in that it comprises:

— an annular carrier (29, 229) arranged around the axis (4, 204) of the support (1, 201), and behind the reference surface (3, 203),

— means (30, 31, 32, 232, 248) for guiding the carrier (29, 229) in translation with respect to the support (1, 201) parallel to the axis (4, 204) of the support (1, 201),

— means (42, 44, 48, 301) defining two stable, determined limit positions of the carrier (29, 229) with respect to the reference surface (3, 203) of the support (1, 201) on the said translation of the carrier (29, 229) with respect to the support (1, 201),

— means (38, 39, 40, 42, 240, 244, 246, 258, 301) for manoeuvring at will the carrier (29, 229) in translation with respect to the support (1, 201) between the said limit positions,

— a plurality of blade carriers (23, 223) of which each is associated with a respective slot (19, 219) and pivotally mounted on the carrier (29, 229) about a respective axis (28, 228) perpendicular to the said plane (20, 220) of the associated slot (19, 219),

– a plurality of blades (22, 222) of which each is fixed to a respective blade carrier (23, 223), the said blade (22, 222) being arranged in the said plane (20, 220) of the associated slot (19, 219) and having across the associated slot (19, 219) an active part (25, 225) in the region of the reference surface (3, 203) the said active part (25, 225) being withdrawn with respect to the reference surface (3, 203) in a first of said limit positions of the said carrier (29, 229) with respect to the reference surface (3, 203) and forming a projection with respect to the reference surface (3, 203) in the second of these two limit positions,

– means (24, 224, 314 to 319, 324) for fixing each of the blades (22, 222) to the associated blade carrier (23, 223),

– means (88, 96, 98, 100, 76, 288, 296, 276) defining two stable, determined limit positions of each blade-carrier (23, 223) pivotally with respect to the carrier (29, 229) defining themselves the said two determined limits, respectively of approach and extension of the active part (25, 225) of the blade (22, 222) with respect to the axis (4, 204) of the support (1, 201),

– means (50, 58, 61, 62, 65, 67, 69, 70, 80, 32, 180, 250, 258, 261, 262, 265, 267, 269, 270, 280, 232, 380) for manoeuvring at will the assembly of blade-carriers (23, 223) pivotally with respect to the carrier (29, 229) about the said pivot axes (28, 228), so that the active parts (25, 225) of the blades (22, 222) are moved together from a first of the said limits, respective of approach to and extension from the axis (4, 204) of the support (1, 201), to the second of these two limits.

14. Apparatus according to claim 13, characterised in that the blades (22, 222) and blade-carriers (23, 223) have mutually disconnectable fixing means (314 to 319, 324).

15. Apparatus according to either claim 13 or claim 14, characterised in that the reference surface (3, 203) is removable and interchangeable at least as regards an annular zone (3a, 203a) situated around the axis (4, 204) of the support (1, 201) and into which the slots (19, 219) open.

16. Apparatus according to any one of claims 13 to 15, characterised in that the reference surface (3, 203) comprises a face of a lens (18, 218) forming an integral part of the support (1, 201) and having the said slots (19, 219).

17. Apparatus according to claims 15 and 16 in combination, characterised in that the lens (18, 218) is removable.

18. Apparatus according to any one of claims 13 to 17, characterised in that the reference face (3, 203) has, internally, a vacuum volume (9, 209, 216) and in that the support has means (111, 211, 302) for connecting the said vacuum volume (9, 209, 216) to a source of vacuum.

19. Apparatus according to claim 18, characterised in that the support (201) limits in a fluid tight manner, around the blades (222), a volume (216) open only towards the reference surface (3, 203) particularly via the said slots (19, 219), and in that it comprises means (302) for connecting the said volume to a source of vacuum.

20. Apparatus according to either claim 18 or claim 19, characterised in that the reference surface (3, 203) has a convexity limited to an annular zone (3a, 203a) situated about the axis (4, 204) of the support (1, 201) and in which the slots (19, 219) open, the said annular zone (3a, 203a) having a shape approximately corresponding to an envelope of arcs of which each is situated in the said plane (20, 220) of a respective slot (19, 219) and centered on the pivot axis (28, 228) of the associated blade-carrier (23, 223) with respect to the carrier (29, 229).

21. Apparatus according to any one of claims 13 to 20, characterised in that the active part (25, 225) of each blade (22, 222) is arranged in accordance with the said plane (20, 220) of the associated slot (19, 219), and in that the guide means (30, 31, 32, 232, 243) for the carrier (29, 229) in translation with respect to the support (1, 201) have means (32, 36, 232, 236) preventing any rotation of the carrier (29, 229) with respect to the support (1, 201) about the axis (4, 204) of the support (1, 201).

22. Apparatus according to any one of claims 13 to 20, characterised in that the active part (25, 225) of each blade (22, 222) is arranged in accordance with a plane inclined with a determined angle with respect to the said plane (20, 220) of the associated slot (19, 219), this angle being identical for all the blades (22, 222), and in that the guide means (30, 31, 32, 232, 243) for the carrier (29, 229) in translation with respect to the support (1, 201) comprises a mutual connection of the carrier (29, 229) and of the support (1, 201) via a helicoidal cam surface centred on the axis (4, 204) of the support (1, 201) and having a helix angle compatible with the said angle determined so that, during the said translation of the carrier (29, 229) with respect to the support (1, 201), the said plane of the active part (25, 225) off each blade (22, 222) remains fixed in respect to the support (1, 201).

23. Apparatus according to either claim 21 or claim 22, characterised in that the means (38, 39, 40, 42) for manoeuvring the carrier (29) in translation with respect to the support (1) comprises a crown (40) carried by the support (1) with the possibility of relative rotation about the axis (4) of the support (1) without possibility of relative translation parallel to the axis (4), mutually engaged complementary screw threads (38, 39), arranged respectively on the carrier (29) and on the said crown (40), and means (42) for manoeuvring at will the said crown (40) in rotation with respect to the support (1), about the axis (4) of the support (1).

24. Apparatus according to either claim 21 or claim 22, characterised in that the means (240, 244, 246, 258, 301) for manoeuvring the carrier (229) in translation with respect to the support (201) comprise a crown (240) carried by the support (201) with the possibility of relative rotation about the axis (204) of the support (201) without possibility of relative translation parallel to this axis (204), the said crown (240) and the carrier (229) being in mutual engagement via the intermediary of at least one cam track (246) carried by one (240) of them and sloped (246b) with reference to the axis (204) of the support (201) and at least one cam follower (244) carried by the other (229) of them opposite the cam track (240), means (258) for ensuring a mutual contact of the cam follower (244) and of the cam track (246), and means (301) for manoeuvring at will the said crown (240) in rotation with respect to the support (201), about the axis (204) of the support (201).

25. Apparatus according to either claim 23 or claim 24, characterised in that means (42, 44, 48, 301) defining two stable limit positions of the carrier (29, 229) with respect to the reference surface (3, 203) of the support (1, 201) comprise abutment means for imposing on the rotation of the crown (40, 240) with respect to the support (1, 201), about the axis (4, 204) of the support (1, 201), limits of rotation corresponding to the said first and second limit positions of the carrier (29, 229) with respect to the reference surface (3, 203).

26. Apparatus according to any one of claims 13 to 25, characterised in that it comprises means (29, 38, 39, 40, 42, 44, 229, 240, 244, 246, 258, 301) for adjusting the said second limit position of the carrier (29, 229) with respect to the reference surface (3, 203).

27. Apparatus according to claims 25 and 26 in combination, characterised in that it comprises means (44, 301) for adjusting at least the one of the limits of rotation of the crown (40, 240) with respect to the support (1, 201) which corresponds to the second limit position of the carrier (29, 229) with respect to the reference surface (3, 203).

28. Apparatus according to any one of claims 13 to 27, characterised in that it comprises means (88, 96, 98, 100, 228, 296) for adjusting the position of the said first limit spacedly with respect to the axis (4, 204) of the support (1, 201).

29. Apparatus according to any one of claims 13 to 28, characterised in that it comprises means (76, 276) for adjusting the position of the said second limit spacedly with respect to the axis of the support.

30. Apparatus according to any one of claims 13 to 29, characterised in that the means (50, 58, 61, 62, 65, 67, 69, 70, 80, 32, 180, 250, 258, 261, 262, 265, 267, 269, 270, 280, 232, 380) for manoeuvring the assembly of blade-carriers (23, 223) pivotally with respect to the carrier (29, 229) comprise:

– an annular sleeve (50, 250) coaxial with the carrier (29, 229),

– means (51, 52, 54, 23, 251, 264, 254, 223) for guiding the sleeve (50, 250) in translation with respect to the carrier (29, 229) parallel to the axis (4, 204) of the support (1, 201) without possibility of relative rotation about the axis (4, 204) of the support (1, 201),

– means (54, 55, 254, 255) for kinematic connection between the sleeve (50, 250) and each of the blade-carriers (23, 223), associating with a translation of the sleeve (50, 250) with respect to the carrier (29, 229) in one or other of two opposite directions parallel to the axis (4, 204) of the support (1, 201) a simultaneous pivoting of the assembly of blade-carriers (23, 223) with respect to the carrier (29, 229) so that the active parts (25, 225) of the blades (22, 222) are displaced respectively towards or away from the axis (4, 204) of the support (1, 201),

– means (58, 61, 62, 65, 67, 69, 70, 80, 32, 180, 258, 261, 262, 265, 267, 269, 270, 280, 232, 380) for manoeuvring at will the sleeve (50, 250) in translation with respect to the carrier (29, 229) in one or other of the said two opposite directions.

31. Apparatus according to claim 30, characterised in that the means (58, 61, 62, 67, 69, 70, 80, 32, 180, 258, 261, 262, 265, 269, 270, 280, 232, 380) for manoeuvring the sleeve (50, 250) in translation with respect to the carrier (29, 229) comprises:

– means (58, 258) for resiliently urging the sleeve (50, 250) in translation in a first of said two opposite directions with respect to the carrier (29, 229),

– reversible means (61, 62, 65, 67, 70, 80, 32, 180, 261, 262, 265, 267, 269, 270, 280, 232, 380) for manoeuvring the sleeve (50, 250) in translation with respect to the carrier (29, 229) in the second of the said two opposite directions,

– means (88, 96, 98, 100, 288, 296) for temporarily fixing the sleeve (50, 250) against a translation under the action of the resilient urging means (58, 258) with respect to the carrier (29, 229), for defining the said first of the said limits respectively of approach and of extension of the active parts (25, 225) of the blades (22, 222) with respect to the axis (4, 204) of the support (1, 201),

– means (76, 276) for defining the said second of the said limits respectively of approach and of extension of the active parts (25, 225) of the blades (22, 222) with respect to the axis (4, 204) of the support (1, 201).

32. Apparatus according to claim 31, characterised in that the reversible means (61, 62, 65, 67, 69, 70, 80, 32, 180, 261, 262, 265, 267, 269, 270, 280, 232, 380) for manoeuvring the sleeve (50, 250) in translation with respect to the carrier (29, 229) in the said second direction comprise a collar (62, 262) carried by the carrier (29, 229) with possibility of relative rotation about the axis (4, 204) of the support (1, 201) without possibility of relative translation parallel to the axis (4, 204); the said collar (62, 262) and the sleeve (50, 250) being in mutual engagement by the intermediary of at least one cam track (61, 261) carried by one (50, 250) of them and sloping (61b, 261b) with reference to the axis (4, 204) of the support (1, 201) and at least one cam follower (65, 265) carried by the other (62, 262) of them opposite the cam track (61, 261), means (58, 258) for assuring a mutual contact of the cam follower (65, 265) and of the cam track (61, 261), and means (70, 80, 32, 180, 270, 280, 232, 380) for manoeuvring at will the said collar (62, 262) in rotation with respect to the carrier (29, 229), about the axis (4, 204) of the support (1, 201),

and in that means (88, 96, 98, 100, 288, 298) for temporarily fixing the sleeve (50, 250) against a translation under the action of the resilient urging means (58, 258) comprise means (88, 96, 98, 100, 288, 296) for temporarily fixing the said collar (62, 262) against a rotation with respect to the carrier (29, 229) about the axis (4, 204) of the support (1, 201).

33. Apparatus according to claim 32, characterised in that the means (88, 96, 98, 100, 288, 296) for temporarily fixing the said collar (62, 262) against the rotation with respect to the carrier (29, 229) about the axis (4, 204) of the support (1, 201) comprise indexation means (96, 98, 100) with a plurality of relative angular positions of temporarily fixing of the said collar (62, 262) with respect to the carrier (29, 229), with respect to the axis (4, 204) of the support (1, 201).

34. Apparatus according to any one of claims 31 to 33, characterised in that the means (76, 276) for defining the said second of the said limits comprise an annular abutment (76, 276), fixed to the support (1, 201), for the blade-carriers (23, 223).

35. Apparatus according to claim 34, characterised in that the said annular abutment (76, 276) and the support (1, 201) have means for mutual removable fixing.

36. Apparatus according to any one of claims 30 to 35, characterised in that the said means (54, 55, 254, 255) of kinematic connection between the sleeve (50, 250) and each of the blade-carriers (23, 223) associate to a translation of the sleeve (50, 250) in the first of the said two opposite directions with respect to the carrier (29, 229), a simultaneous pivoting of the assembly of the blade-carriers (23, 223) with respect to the carrier (29, 229) such that the active parts (25, 225) of the blades (22, 222) are displaced from the said limit of approach with respect to the axis (4, 204) of the support (1, 201), constituting the said first limit, to the said limit of extension with respect to the axis (4, 204) of the support (1, 201), constituting the said second limit.

**Patentansprüche**

1. Chirurgische Vorrichtung für radiale Keratotomie mit einem Träger (1, 201), welcher aufweist:
- eine Achse (4, 204) für den Träger (1, 201),
- eine konkave Bezugsoberfläche (3, 203), welche die Achse (4, 204) des Trägers (1, 201) schneidet und geeignet ist, sich in einer Position, in der die Achse (4, 204) des Trägers (1, 201) mit der optischen Achse (5, 205) eines Auges (2, 202) übereinstimmt, an die Hornhaut (2a, 202a) des Auges anzuschmiegen,
- eine Mehrzahl von Schlitzen (19, 219) von denen jeder sich in einer entsprechenden, die Achse (4, 204) des Trägers (1, 201) einschliessenden Ebene (20, 220) angeordnet ist und in einer Zone (3a, 203a) der Bezugsoberfläche (3, 203) mündet, wobei jeder Schlitz (19, 219) den Durchtritt einer Klinge (22, 222) ermöglicht, derart, dass die betreffende Klinge (22, 222) einen aktiven Abschnitt (25, 225) aufweist, welcher einen gegenüber der Bezugsoberfläche (3, 203) vorgegebenen Vorsprung bildet und sich in der besagten Ebene (20, 220) des Schlitzes (19, 219) zwischen zwei vorgegebenen Endstellungen bewegen kann, nämlich jeweils einer Annäherungsendstellung und einer Entfernungsendstellung des aktiven Abschnitts (25, 225) der Klinge (22, 222) bezüglich der Achse (4, 204) des Trägers (1, 201),

dadurch gekennzeichnet, dass der Träger (1, 201) eine Mehrzahl von Klingen (20, 220), von denen jede mit einem entsprechenden Schlitz (19, 219) verbunden ist, sowie Einrichtungen (50, 58, 61, 62, 65, 67, 69, 70, 80, 32, 180, 250, 258, 261, 262, 265, 267, 269, 270, 280, 232, 380), die gegenüber der Bezugsoberfläche zurückgesetzt angeordnet sind, trägt, um eine gleichzeitige Verstellung der Klingen (22, 222) bezüglich des Trägers (1, 201) in den entsprechend zugehörigen Schlitzen und in deren entsprechender Ebene (20, 220) hervorzurufen, und zwar von einer jeweils gleichen ersten unter den entsprechenden beiden vorgegebenen Endstellungen in die jeweils zweite der beiden entsprechend vorgegebenen Endstellungen, während ein aktiver Abschnitt (25, 225) einer jeden Klinge (22, 222) um eine entsprechend vorgegebene Strecke aus der Bezugsoberfläche (3, 203) hervortritt.

2. Vorrichtung nach Anspruch 1, dadurch gekennzeichnet, dass sie Einrichtungen (29, 38, 39, 40, 42, 229, 240, 244, 246, 258, 301) zum reversiblen Einziehen des aktiven Abschnittes (25, 225) der Klingen (22, 222) bezüglich der Bezugsoberfläche (3, 203) umfasst.

3. Vorrichtung nach Anspruch 1 oder 2, dadurch gekennzeichnet, dass die Klingen (22, 222) abnehmbar und austauschbar sind.

4. Vorrichtung nach einem der Ansprüche 1 bis 3, dadurch gekennzeichnet, dass die Bezugsoberfläche (2, 202) abnehmbar und austauschbar ist, zumindest, was eine ringförmige Zone (3a, 203a) betrifft, welche um die Achse (4, 204) des Trägers (1, 201) herum angeordnet ist und in welcher die Schlitze (19, 219) münden.

5. Vorrichtung nach einem der Ansprüche 1 bis 4, dadurch gekennzeichnet, dass die Bezugsoberfläche (3, 203) eine Oberfläche einer Linse (18, 218) umfasst, welche einen zum Träger (1, 201) gehörenden Teil bildet und die besagten Schlitze (19, 219) aufweist.

6. Vorrichtung nach einem der Ansprüche 1 bis 5, dadurch gekennzeichnet, dass sie Einrichtungen (9, 10, 111, 209, 210, 311, 216, 302) zum pneumatischen Anlagern der Hornhaut (2a, 202a) an der Bezugsoberfläche (3, 203) besitzt.

7. Vorrichtung nach Anspruch 6, dadurch gekennzeichnet, dass der Träger (201) um die Klingen (222) herum ein Volumen (216) in dichter Weise begrenzt, welches ausschliesslich zur Bezugsoberfläche (3, 203) hin offen ist, insbesondere über die besagten Schlitze (19, 219), und dass sie Einrichtungen (302) zur Verbindung dieses Volumens mit einer Ansaugquelle besitzt.

8. Vorrichtung nach einem der Ansprüche 6 und 7, dadurch gekennzeichnet, dass die Bezugsoberfläche (3, 203) eine Konvexität besitzt, welche auf eine ringförmige Zone (3a, 203a) begrenzt ist, die um die Achse (4, 204) des Trägers (1, 201) herum gelegen ist und in welcher die Schlitze (19, 219) münden.

9. Vorrichtung nach einem der Ansprüche 1 bis 8, dadurch gekennzeichnet, dass sie Einrichtungen (29, 38, 39, 40, 42, 44, 229, 240, 244, 246, 258, 301) zur Einstellung des besagten Vorsprungs gegenüber der Bezugsoberfläche (3, 203) besitzt.

10. Vorrichtung nach einem der Ansprüche 1 bis 9, dadurch gekennzeichnet, dass sie Einrichtungen (88, 96, 98, 100, 288, 296) besitzt, um die Position der besagten ersten Endstellung in der Entfernung gegenüber der Achse (4, 204) des Trägers (1, 201) einzustellen.

11. Vorrichtung nach einem der Ansprüche 1 bis 10, dadurch gekennzeichnet, dass sie Einrichtungen (76, 276) besitzt, um die Position der besagten zweiten Endstellung in der Entfernung gegenüber der Achse des Trägers einzustellen.

12. Vorrichtung nach einem der Ansprüche 1 bis 11, dadurch gekennzeichnet, dass die besagte erste und die besagte zweite Endstellung jeweils die Endstellung für die Annäherungsposition und die Endstellung für die Entfernungsposition des aktiven Abschnitts (25, 225) einer jeden Klinge (22, 222) bezüglich der Achse (4, 204) des Trägers (1, 201) sind.

13. Vorrichtung für radiale Keratotomie, mit einem Träger (1, 201), welcher aufweist:
  – eine Achse (4, 204) für den Träger (1, 201),
  – eine konkave Bezugsoberfläche (3, 203), welche die Achse (4, 204) für den Träger (1, 201) schneidet und geeignet ist, sich in einer Position, in der die Achse (4, 204) des Trägers (1, 201) mit der optischen Achse (5, 205) eines Auges (2, 202) übereinstimmt, an die Hornhaut (2a, 202a) des Auges anzuschmiegen,
  – eine Mehrzahl von Schlitzen (19, 219), von denen jeder in einer entsprechenden, die Achse (4, 204) des Trägers (1, 201) einschliessenden Ebene (20, 220) angeordnet ist und in einer Zone (3a, 203a) der Bezugsoberfläche (3, 203) mündet, wobei jeder Schlitz (19, 219) den Durchtritt einer Klinge (22, 220) ermöglicht, derart, dass die betreffende Klinge (22, 222) einen aktiven Abschnitt (25, 225) aufweist, welcher einen gegenüber der Bezugsoberfläche (3, 203) vorgegebenen Vorsprung bildet und sich in der besagten Ebene (20, 220) des Schlitzes (19, 219) zwischen zwei vorgegebenen Endstellungen bewegen kann, nämlich jeweils einer Annäherungsendstellung und einer Entfernungsendstellung des aktiven Abschnitts (25, 225) der Klinge (22, 222) bezüglich der Achse (4, 204) des Trägers (1, 201),
  dadurch gekennzeichnet, dass die Vorrichtung aufweist:
  – einen um die Achse (4, 204) des Trägers (1, 201) angeordneten, gegenüber der Bezugsoberfläche (3, 203) zurückgesetzten ringförmigen Schlitten (29, 229),
  – Einrichtungen (30, 31, 32, 232, 248) zur Führung des Schlittens (29, 229) für eine Verschiebung bezüglich des Trägers (1, 201) parallel zur Achse (4, 204) des Trägers (1, 201),
  – Einrichtungen (42, 44, 48, 301), welche zwei stabile, vorgegebene Endpositionen des Schlittens (29, 229) bezüglich der Bezugsoberfläche (3, 203) des Trägers (1, 201) bei der besagten Verschiebung des Schlittens (29, 229) gegenüber dem Träger (1, 201) definieren,

  – Einrichtungen (38, 39, 40, 42, 240, 244, 246, 258, 301) zur beliebigen Betätigung des Schlittens (29, 229) für eine Verschiebung gegenüber dem Träger (1, 201) zwischen den besagten Endpositionen,
  – eine Mehrzahl von Klingenträgern (23, 223), von denen jeder mit einem entsprechenden Schlitz (19, 219) verbunden und auf dem Schlitten (29, 229) um eine jeweilige, zur Ebene (20, 220) des zugehörigen Schlitzes (19, 219) senkrechte Achse (28, 228) drehbar angebracht ist,
  – eine Mehrzahl von Klingen (22, 222), von denen jede fest mit einem entsprechenden Klingenträger (23, 223) verbunden ist, wobei die besagte Klinge (22, 222) jeweils entlang der besagten Ebene (20, 220) des zugeordneten Schlitzes (19, 219) angeordnet ist und durch den zugeordneten Schlitz (19, 219) hindurch jeweils einen aktiven Abschnitt (25, 225) in der Nähe der Bezugsoberfläche (3, 203) aufweist, wobei der besagte aktive Abschnitt (25, 225) in einer ersten der besagten zwei Endstellungen des Schlittens (29, 229) bezüglich der Bezugsoberfläche (3, 203) gegenüber dieser Bezugsoberfläche (3, 203) versenkt ist und in der zweiten dieser beiden Endstellungen gegenüber der Bezugsoberfläche (3, 203) vorspringt,
  – Einrichtungen (24, 224, 314–319, 324) zur Befestigung einer jeden der Klingen (22, 222) mit dem zugeordneten Klingenträger (23, 223),
  – Einrichtungen (88, 96, 98, 100, 76, 288, 296, 276), welche zwei stabile, vorgegebene Endpositionen jeden Klingenträger (23, 223) in seiner Drehung gegenüber dem Schlitten (29, 229) definieren, wobei diese selbst die besagten zwei vorgegebenen Endstellungen definieren, nämlich jeweils für die Annäherungsposition und für die Entfernungsposition des aktiven Abschnitts (25, 225) der entsprechenden Klinge (22, 222) bezüglich der Achse (4, 204) des Trägers (1, 201),
  – Einrichtungen (50, 58, 61, 62, 65, 67, 69, 70, 80, 32, 180, 250, 258, 261, 262, 265, 267, 269, 270, 280, 232, 380) zur beliebigen Betätigung aller Klingenträger (23, 223) zur Drehung gegenüber dem Schlitten (29, 229) um die besagten Drehachsen (28, 228), derart, dass die aktiven Abschnitte (25, 225) der Klingen (22, 222) sich gleichzeitig von einer ersten der beiden Endstellungen, nämlich der Annäherungs- und der Entfernungsstellung bezüglich der Achse (4, 204) des Trägers (1, 201) zu der zweiten unter den beiden Endstellungen bewegen.

14. Vorrichtung nach Anspruch 13, dadurch gekennzeichnet, dass die Klingen (22, 222) und die Klingenträger (23, 223) Einrichtungen zur gegenseitigen lösbaren Verbindung (314–319, 324) aufweisen.

15. Vorrichtung nach einem der Ansprüche 13 und 14, dadurch gekennzeichnet, dass die Bezugsoberfläche (2, 202) abnehmbar und austauschbar ist, zumindest, soweit es eine ringförmige Zone (3a, 203a) betrifft, welche um die Achse (4, 204) des Trägers (1, 201) herum gelegen ist und in welcher die Schlitze (19, 219) münden.

16. Vorrichtung nach einem der Ansprüche 13 bis 15, dadurch gekennzeichnet, dass die Bezugsoberfläche (3, 203) eine Oberfläche einer Linse (18, 218) besitzt, welche einen zum Träger (1, 201) gehörenden Abschnitt bildet und die besagten Schlitze (19, 219) aufweist.

17. Vorrichtung nach den Ansprüchen 15 und 16 in Kombination, dadurch gekennzeichnet, dass die Linse (18, 19) abnehmbar ist.

18. Vorrichtung nach einem der Ansprüche 13 bis 17, dadurch gekennzeichnet, dass die Bezugsoberfläche (3, 203) vertieft einen Absaugraum (9, 209, 216) aufweist und dass der Träger Einrichtungen (111, 311, 302) zur Verbindung des besagten Absaugraumes (9, 209, 216) mit einer Vakuumquelle besitzt.

19. Vorrichtung nach Anspruch 18, dadurch gekennzeichnet, dass der Träger (201) um die Klingen (222) herum jeweils in dichter Weise einen ausschliesslich zur Bezugsoberfläche (3, 203), insbesondere über die besagten Schlitze (19, 219), offenen Raum (216) begrenzt, und dass sie Einrichtungen (302) zur Verbindung des besagten Raumes mit einer Absaugquelle besitzt.

20. Vorrichtung nach einem der Ansprüche 18 und 19, dadurch gekennzeichnet, dass die Bezugsoberfläche (3, 203) eine auf eine ringförmige Zone (3a, 203a), welche um die Achse (4, 204) des Trägers (1, 201) herum gelegen ist und in welcher die Schlitze (19, 219) münden, begrenzte Konvexität aufweist, wobei die ringförmige Zone (3a, 203a) eine Form aufweist, welche annähernd einer Hüllkurve von Bögen entspricht, von denen jeder in der besagten Ebene (20, 220) eines entsprechenden Schlitzes (19, 219) liegt und zu der Drehachse (28, 228) des bezüglich des Schlitzes (29, 229) zugeordneten Klingenträgers (23, 223) ausgerichtet ist.

21. Vorrichtung nach einem der Ansprüche 13 bis 20, dadurch gekennzeichnet, dass der aktive Abschnitt (25, 225) einer jeden Klinge (22, 222) in der Ebene (20, 220) des zugehörigen Schlitzes (19, 219) angeordnet ist und dass die Einrichtungen (30, 31, 32, 232, 243) zur Führung des Schlittens (29, 229) zur Verschiebung gegenüber dem Träger (1, 201) Einrichtungen (32, 36, 232, 236) besitzen, welche jegliche Drehung des Schlittens (29, 229) bezüglich des Trägers (1, 201) um die Achse (4, 204) des Trägers (1, 201) verhindern.

22. Vorrichtung nach einem der Ansprüche 13 bis 20, dadurch gekennzeichnet, dass der aktive Abschnitt (25, 225) einer jeden Klinge (22, 222) in einer um einen vorbestimmten Winkel gegenüber der besagten Ebene (20, 220) des zugehörigen Schlitzes (19, 219) geneigten Ebene angeordnet ist, wobei dieser Winkel identisch für alle Klingen (22, 222) ist, und dass die Einrichtungen (30, 31, 32, 232, 243) zur Führung des Schlittens (29, 229) zur Verschiebung gegenüber dem Träger (1, 201) eine wechselseitige Verbindung des Schlittens (29, 229) und des Trägers (1, 201) über eine Schraubenkurvenfläche besitzen, welche nach der Achse (4, 204) des Trägers (1, 201) ausgerichtet ist und einen Steigungswinkel besitzt, welcher mit dem besagten vorgegebenen Winkel abgestimmt ist, damit während der besagten Verschiebung des Schlittens (29, 229) bezüglich des Trägers (1, 201) die besagte Ebene des aktiven Abschnitts (25, 225) einer jeden Klinge (22, 222) bezüglich des Trägers (1, 201) fest bleibt.

23. Vorrichtung nach einem der Ansprüche 21 und 22, dadurch gekennzeichnet, dass die Einrichtungen (38, 39, 40, 42) zur Betätigung des Schlittens (29) zur Verschiebung gegenüber dem Träger (1) einen Ring (40), welcher von dem Träger (1) mit der Möglichkeit einer Relativdrehung um die Achse (4) des Trägers (1) ohne die Möglichkeit einer Relativverschiebung parallel zu dieser Achse (4) getragen wird, komplementäre Rippen (38, 39), welche in wechselseitigem Eingriff und jeweils auf dem Schlitten (29) und auf dem besagten Ring (40) angebracht sind, und Einrichtungen (42) zur beliebigen Betätigung des Rings (40) zur Drehung gegenüber dem Träger (1) um die Achse (4) des Trägers besitzen.

24. Vorrichtung nach einem der Ansprüche 21 und 22, dadurch gekennzeichnet, dass die Einrichtungen (240, 244, 246, 258, 301) zur Betätigung des Schlittens (229) in Translationsbewegung bezüglich des Trägers (201) einen von dem Träger (201) getragenen Ring (240) mit der Möglichkeit einer Relativdrehung um die Achse (204) des Trägers (201) ohne die Möglichkeit einer relativen Translationsbewegung parallel zu dieser Achse (204) umfassen, wobei der besagte Ring (240) und der Schlitten (229) in gegenseitigem Eingriff stehen, und zwar mittels mindestens einer Kurvenbahn (246), welche von dem einen (240) von ihnen getragen wird und bezüglich der Achse (204) des Trägers (201) geneigt (246b) ist, und mittels mindestens eines Kurvenabtastelementes (244), welches von dem anderen (229) unter diesen beiden Teilen im Hinblick auf die Kurvenbahn (240) getragen wird, dass diese Einrichtungen weiterhin Mittel (258) zur Sicherstellung eines wechselseitigen Kontaktes zwischen dem Kurvenabtastelement (244) und der Kurvenbahn (246) sowie Mittel (301) zur beliebigen Betätigung des Ringes (240) für eine Drehung bezüglich des Trägers (201) um die Achse (204) des Trägers (201) besitzen.

25. Vorrichtung nach einem der Ansprüche 23 und 24, dadurch gekennzeichnet, dass die zwei stabilen Endstellungen des Schlittens (29, 229) bezüglich der Bezugsoberfläche (3, 203) des Trägers (1, 201) definierenden Einrichtungen (42, 44, 48, 301) Anschlagelemente besitzen, um für die Drehung des Ringes (40, 240) bezüglich des Trägers (1, 201) um die Achse (4, 204) des Trägers (1, 201) Drehendstellungen entsprechend der besagten ersten und der besagten zweiten Endstellung des Schlittens (29, 229) bezüglich der Bezugsoberfläche (3, 203) festzulegen.

26. Vorrichtung nach einem der Ansprüche 13 bis 25, dadurch gekennzeichnet, dass sie Einrichtungen (29, 38, 39, 40, 42, 44, 229, 240, 244, 246, 258, 301) besitzt, um die besagte zweite Endstellung des Schlittens (29, 229) bezüglich der Bezugsoberfläche (3, 203) einzustellen.

27. Vorrichtung nach den Ansprüchen 25 und 26, in Kombination, dadurch gekennzeichnet, dass sie Einrichtungen (44, 301) besitzt, um mindestens diejenige der Drehendstellungen des Ringes (40, 240) bezüglich des Trägers (1, 201) einzustellen, welche der zweiten Endstellung des Schlittens (29, 229) bezüglich der Bezugsoberfläche (3, 203) entspricht.

28. Vorrichtung nach einem der Ansprüche 13 bis 27, dadurch gekennzeichnet, dass sie Einrichtungen (88, 96, 98, 100, 228, 296) aufweist, um die Position der besagten ersten Entfernungsendstellung bezüglich der Achse (4, 204) des Trägers (1, 201) einzustellen.

29. Vorrichtung nach einem der Ansprüche 13 bis 28, dadurch gekennzeichnet, dass sie Einrichtungen (76, 276) besitzt, um die Position der besagten zweiten Entfernungsendstellung bezüglich der Achse des Trägers einzustellen.

30. Vorrichtung nach einem der Ansprüche 13 bis 29, dadurch gekennzeichnet, dass die Einrichtungen (50, 58, 61, 62, 65, 67, 69, 70, 80, 32, 180, 250, 258, 261, 262, 265, 267, 269, 270, 280, 232, 380) zur Betätigung aller Klingenträger (23, 223) zur Drehung gegenüber dem Schlitten (29, 229) aufweisen:

– eine zum Schlitten (29, 229) koaxiale ringförmige Manschette (50, 250),

– Einrichtungen (51, 52, 54, 23, 251, 264, 254, 223) zur Führung der Manschette (50, 250) in Translationsbewegung bezüglich des Schlittens (29, 229) parallel zur Achse (4, 204) des Trägers (1, 201) ohne die Möglichkeit einer relativen Drehung um die Achse (4, 204) des Trägers (1, 201),

– Einrichtungen (54, 55, 254, 255) zur Zwangsverbindung zwischen der Manschette (50, 250) und einem jeden der Klingenträger (23, 223), welche mit einer Translationsbewegung der Manschette (50, 250) bezüglich des Schlittens (29, 229) in dem einen oder in dem anderen der beiden entgegengesetzten Richtungen parallel zur Achse (4, 204) des Trägers (1, 201) eine gleichzeitige Drehung aller Klingenträger (23, 223) bezüglich des Schlittens (29, 229) derart verbinden, dass die aktiven Abschnitte (25, 225) der Klingen (22, 222) sich jeweils entsprechend bei der Annäherung oder Entfernung bezüglich der Achse (4, 204) des Trägers (1, 201) bewegen,

– und Einrichtungen (58, 61, 62, 65, 67, 69, 70, 80, 32, 180, 258, 261, 262, 265, 267, 269, 270, 280, 232, 380) zur beliebigen Betätigung der Manschette (50, 250) bei der Translationsbewegung bezüglich des Schlittens (29, 229) in der einen oder der anderen der beiden entgegengesetzten Richtungen.

31. Vorrichtung nach Anspruch 30, dadurch gekennzeichnet, dass die Einrichtungen (58, 61, 62, 65, 67, 69, 70, 80, 32, 180, 258, 261, 262, 265, 267, 269, 270, 280, 232, 380) zur Betätigung der Manschette (50, 250) zu einer Translationsbewegung bezüglich des Schlittens (29, 229) aufweisen:

– Einrichtungen (58, 158) zur elastischen Vorspannung der Manschette (50, 250) für die Translationsbewegung in einer ersten der beiden entgegengesetzten Richtungen bezüglich des Schlittens (29, 229),

– reversible Einrichtungen (61, 62, 65, 67, 69, 70, 80, 32, 180, 261, 262, 265, 267, 269, 270, 280, 232, 380) zur Betätigung der Manschette (50, 250) in Translationsbewegung bezüglich des Schlittens (29, 229) in der zweiten der besagten zwei entgegengesetzten Richtungen,

– Einrichtungen (88, 96, 98, 100, 288, 296) zur vorläufigen Festlegung der Manschette (50, 250) entgegen einer Translationsbewegung unter der Einwirkung der elastischen Vorspanneinrichtungen (58, 258) bezüglich des Schlittens (29, 229), um die besagte erste unter den beiden Endstellungen hinsichtlich Annäherung und Entfernung der aktiven Abschnitte (25, 225) der Klingen (22, 222) bezüglich der Achse (4, 204) des Trägers (1, 201) zu definieren,

– Einrichtungen (76, 276), um die besagte zweite unter den Endstellungen bezüglich Annäherung und Entfernung der aktiven Abschnitte (25, 225) der Klingen (22, 222) bezüglich der Achse (4, 204) des Trägers (1, 201) zu definieren.

32. Vorrichtung nach Anspruch 31, dadurch gekennzeichnet, dass die reversiblen Einrichtungen (61, 62, 65, 67, 69, 70, 80, 32, 180, 261, 262, 265, 267, 269, 270, 280, 232, 380) zur Betätigung der Manschette (50, 250) in Translationsbewegung bezüglich des Schlittens (29, 229) in der besagten zweiten Richtung einen von dem Schlitten (29, 229) getragenen Ring (62, 262) mit der Möglichkeit einer Relativdrehung um die Achse (4, 204) des Trägers (1, 201) und ohne die Möglichkeit einer relativen Translationsbewegung parallel zu dieser Achse (4, 204) besitzen, wobei dieser Ring (62, 262) und die Manschette (50, 250) in wechselseitigem Eingriff stehen, und zwar mittels mindestens einer Kurvenbahn (61, 261), welche von dem einen (50, 250) von ihnen getragen wird und bezüglich der Achse (4, 204) des Trägers (1, 201) geneigt (61b, 261b) ist, und mittels mindestens eines Kurvenabtastelements (65, 265), welche von dem anderen (62, 262) in Hinblick auf die Kurvenbahn (61, 261) getragen wird, dass diese Einrichtungen weiterhin Mittel (58, 258) zur Sicherstellung eines wechselseitigen Kontaktes des Kurvenabtastelementes (65, 265) und der Kurvenbahn (61, 261) sowie Mittel (70, 80, 32, 81, 270, 280, 232, 380) zur beliebigen Betätigung des genannten Ringes (62, 262) zur Drehung bezüglich des Schlittens (29, 229) um die Achse (4, 204) des Trägers (1, 201) aufweisen, und dass die Einrichtungen (88, 96, 98, 100, 288, 298) zur vorläufigen Festlegung der Manschette (50, 250) entgegen einer Translationsbewegung unter der Einwirkung der Einrichtungen (58, 258) zur elastischen Vorspannung Mittel (88, 96, 98, 100, 288, 296) zur vorläufigen Festlegung des besagten Ringes (62, 262) entgegen einer Rotationsbewegung bezüglich des Schlittens (29, 229) um die Achse (4, 204) des Trägers (1, 201) aufweisen.

33. Vorrichtung nach Anspruch 32, dadurch gekennzeichnet, dass die Mittel (88, 96, 98, 100, 288, 296) zur vorläufigen Festlegung des besagten Ringes (62, 262) entgegen einer Drehbewegung bezüglich des Schlittens (29, 229) um die Achse (4, 204) des Trägers (1, 201) Mittel (96, 98, 100) zur Indexation einer Mehrzahl von relativen Winkelpositionen für die vorläufige Festlegung des besagten Ringes (62, 262) gegenüber dem Schlitten (29, 229) bezüglich der Achse (4, 204) des Trägers (1, 201) aufweisen.

34. Vorrichtung nach einem der Ansprüche 31 bis 33, dadurch gekennzeichnet, dass die Mittel (76, 276) zur Festlegung der besagten zweiten unter den Endstellungen einen ringförmigen, mit dem Träger (1, 201) fest verbundenen Anschlag (76, 276) für die Klingenträger (23, 223) aufweisen.

35. Vorrichtung nach Anspruch 34, dadurch gekennzeichnet, dass der besagte ringförmige Anschlag (76, 276) und der Träger (1, 201) Einrichtungen zur gegenseitigen lösbaren Befestigung aufweisen.

36. Vorrichtung nach einem der Ansprüche 30 bis 35, dadurch gekennzeichnet, dass die besagten Einrichtungen (54, 55, 254, 255) zur Zwangsverbindung zwischen der Manschette (50, 250) und einem jeden der Klingenträger (23, 223) mit einer Translationsbewegung der Manschette (50, 250) in der ersten der beiden entgegengesetzten Richtungen bezüglich des Schlittens (29, 229) eine gleichzeitige Drehbewegung der Anordnung von Klingenträgern (23, 223) bezüglich des Schlittens (29, 229) derart verbinden, dass die aktiven Abschnitte (25, 225) der Klingen (22, 222) sich von der besagten Annäherungsendstellung bezüglich der Achse (4, 204) des Trägers (1, 201), welche die besagte erste Endstellung bildet, zu der besagten Entfernungsendstellung bezüglich der Achse (4, 204) des Trägers (1, 201), welche die besagte zweite Endstellung bildet, bewegen.

FIG_1

FIG. 2

FIG_3

FIG.4

FIG.5